# FASCICULE DE BREVET EUROPEEN

(11) **EP 1 332 217 B1**
(45) Date de publication et mention de la délivrance du brevet: **21.04.2010**
(21) Numéro de dépôt: 01993689.7
(22) Date de dépôt: 12.11.2001
(51) Int. Cl.: C12N 15/55, C12N 9/16, C12N 15/63, C12N 5/10, C12N 15/82, C12N 1/20, C12P 21/00, A23K 1/165

(54) **NOUVELLES PHYTASES BACTERIENNES ET PROCEDE DE PRODUCTION DE CES PHYTASES**
NEUE BAKTERIELLE PHYTASEN UND VERFAHREN ZUR HERSTELLUNG DERSELBEN
NOVEL BACTERIAL PHYTASES AND METHOD FOR PRODUCING SAME

(30) Priorité: 10.11.2000 FR 0014448
(43) Date de publication de la demande: 06.08.2003
(73) Titulaire: Adisseo France S.A.S., 92160 Antony (FR)
(72) Inventeur: RAVOT, Gilles, F-30900 Nimes (FR)
(74) Mandataire: Hinterberg, Katherine
(86) Numéro de dépôt international: PCT/FR2001/003527
(87) Numéro de publication internationale: WO 2002/038774

(56) Documents cités:
- EP-A- 0 955 362
- EP-A- 0 960 934
- WO-A-99/48380
- DATABASE SWALL [en ligne] 1 novembre 1996 (1996-11-01) REILLY ET AL.: "Acid phosphatase precursor" retrieved from EBI Database accession no. Q47936 XP002205562 -& DATABASE EM_PRO [en ligne] EMBL; 4 mars 1995 (1995-03-04) REILLY ET AL.: "Francisella tularensis acid phosphatase (acpA) gene, complete cds." retrieved from EBI, accession no. FTACDPHS Database accession no. L39831 XP002205563 & J BIOL CHEM, vol. 271, 1996, pages 0973-10983,
- DATABASE WPI Section Ch, Week 199519 Derwent Publications Ltd., London, GB; Class B04, AN 1995-143836 XP002171298 & JP 07 067635 A (AMANO PHARM KK), 14 mars 1995 (1995-03-14) cité dans la demande
- PEN J ET AL: "PHYTASE-CONTAINING TRANSGENIC SEEDS AS A NOVEL FEED ADDITIVE FOR IMPROVED PHOSPHORUS UTILIZATION" BIO/TECHNOLOGY, NATURE PUBLISHING CO. NEW YORK, US, vol. 11, no. 7, 1 juillet 1993 (1993-07-01), pages 811-814, XP002026203 ISSN: 0733-222X
- DATABASE BIOSIS [en ligne] BIOSCIENCES INFORMATION SERVICE, PHILADELPHIA, PA, US; 1993 KISHIMOTO NORIAKI ET AL: "Acidiphilium aminolytica, new species: An acidophilic chemoorganotrophic bacterium isolated from acidic mineral environment." Database accession no. PREV199396083022 XP002171297 & CURRENT MICROBIOLOGY, vol. 27, no. 3, 1993, pages 131-136, ISSN: 0343-8651
- DATABASE WPI Section Ch, Week 199541 Derwent Publications Ltd., London, GB; Class D16, AN 1995-315923 XP002171299 & JP 07 213281 A (AMANO PHARM KK), 15 août 1995 (1995-08-15)

### LISTE DE SEQUENCES

<110> Aventis Animal Nutrition SA
<120> Nouvelles phytases bactériennes et procédé de production de ces phytases
<130> PM 00080
<140>
   <141>
<150> FR 0014448
   <151> 2000-11-10
<160> 11
<170> PatentIn Ver. 2.1
<210> 1
   <211> 1665
   <212> ADN
   <213> g. Acidocella
<220>
   <221> CDS
   <222> (1)..(1665)
<400> 1
   atg aat atc cgc cgg gct ctt ttg tgc gcc tcc atg atg gtc tcg tcg    48
   Met Asn Ile Arg Arg Ala Leu Leu Cys Ala Ser Met Met Val Ser Ser
    1               5                  10                  15
   atc acc ccc gcg gcg gcg cag acc gcc agc ctc ggc gcc acc gcg ccc    96
   Ile Thr Pro Ala Ala Ala Gln Thr Ala Ser Leu Gly Ala Thr Ala Pro
               20                  25                  30
   gcc tcc gcc agc gcc gca gct tcc tac acc gac gcg ctg ccg acc gcg    144
   Ala Ser Ala Ser Ala Ala Ala Ser Tyr Thr Asp Ala Leu Pro Thr Ala
           35                  40                  45
   acg ccg atc aag cat ctg gtc atc atc tat ggc gaa aac gtc tcc ttc    192
   Thr Pro Ile Lys His Leu Val Ile Ile Tyr Gly Glu Asn Val Ser Phe
       50                  55                  60
   gac cat tat ttc gcc acc tac ccg aag gcc acc aac ccg gcc ggt gag    240
   Asp His Tyr Phe Ala Thr Tyr Pro Lys Ala Thr Asn Pro Ala Gly Glu
   65                  70                  75                  80
   ccg gcc ttc cat ggc agc ctg cat ggc cag aaa atc gat aac ctg gtc    288
   Pro Ala Phe His Gly Ser Leu His Gly Gln Lys Ile Asp Asn Leu Val
                   85                  90                  95
   acc gcc aat ctg ctg agc aat aac ccg aac ttc acc aac gag aaa aac    336
   Thr Ala Asn Leu Leu Ser Asn Asn Pro Asn Phe Thr Asn Glu Lys Asn
               100                 105                 110
   ggc gcc ggg gcc gcc aac ccg ttc cgg ctg gac cgc acc cag gcc gcc    384
   Gly Ala Gly Ala Ala Asn Pro Phe Arg Leu Asp Arg Thr Gln Ala Ala
           115                 120                 125
   acc gcc gac cag aac cat gcc tac aag ccc gag cag gaa gcc tac gat    432
   Thr Ala Asp Gln Asn His Ala Tyr Lys Pro Glu Gln Glu Ala Tyr Asp
       130                 135                 140
   aac ggc aag atg gac ctg ttc ccc aaa tat acc ggc aag gcc agc gcc    480
   Asn Gly Lys Met Asp Leu Phe Pro Lys Tyr Thr Gly Lys Ala Ser Ala
   145                 150                 155                 160
   ggc ggc gcc ggc gcc ttc ggc acc aag ggc cag gtg atg ggc tat ttc      528
   Gly Gly Ala Gly Ala Phe Gly Thr Lys Gly Gln Val Met Gly Tyr Phe
                   165                 170                 175
   gac ggc aac acc gtc acc gcc ctc tgg aac tac gcc cag ggc tac gcg      576
   Asp Gly Asn Thr Val Thr Ala Leu Trp Asn Tyr Ala Gln Gly Tyr Ala
               180                 185                 190
   atg agc gac aat gcc tgg acc gac acc tac ggc ccc tcc acc ccc ggc      624
   Met Ser Asp Asn Ala Trp Thr Asp Thr Tyr Gly Pro Ser Thr Pro Gly
           195                 200                 205
   gcg ctg gcg gtg gtc tcc ggc cag acc aac ggc gcc gtg ccg gtg ctc      672
   Ala Leu Ala Val Val Ser Gly Gln Thr Asn Gly Ala Val Pro Val Leu
       210                 215                 220
   ggc acc tcg ccc aac ctg aac ccg gac ggc cag ggc ggc ttc acc gat      720
   Gly Thr Ser Pro Asn Leu Asn Pro Asp Gly Gln Gly Gly Phe Thr Asp
   225                 230                 235                 240
   gac ggc gat atc gac ccc gcc tac gat gtc tgc tcc agc aag aag gtc      768
   Asp Gly Asp Ile Asp Pro Ala Tyr Asp Val Cys Ser Ser Lys Lys Val
                   245                 250                 255
   acc ttc aag atg gag ggc cag aat atc ggc gat ctg ctg aac gcc aag      816
   Thr Phe Lys Met Glu Gly Gln Asn Ile Gly Asp Leu Leu Asn Ala Lys
               260                 265                 270
   aaa gtc acc tgg ggc ggt ttc atg ggt ggc ttc gat ctc ggc ctc acc      864
   Lys Val Thr Trp Gly Gly Phe Met Gly Gly Phe Asp Leu Gly Leu Thr
           275                 280                 285
   aac ccc aac ggc acc acc ggc tgc aag cgc tcc agc ttc gcc acc gcg      912
   Asn Pro Asn Gly Thr Thr Gly Cys Lys Arg Ser Ser Phe Ala Thr Ala
       290                 295                 300
   gtc gat gcc ccc acc gcc gac tat atc ccg cac cat aac tgg ttc cag      960
   Val Asp Ala Pro Thr Ala Asp Tyr Ile Pro His His Asn Trp Phe Gln
   305                 310                 315                 320
   tat tac gcc tcc acc gcc aac tac acc cat gcc cgc ccg gcg gcg ctg      1008
   Tyr Tyr Ala Ser Thr Ala Asn Tyr Thr His Ala Arg Pro Ala Ala Leu
                   325                 330                 335
   gac gat atc ggc tac agc ttc gcg cca tcc ggc gcc gcc gat ccg gcc      1056
   Asp Asp Ile Gly Tyr Ser Phe Ala Pro Ser Gly Ala Ala Asp Pro Ala
               340                 345                 350
   aac cat gaa tat gat ctg aag gat ttc gag gcg gcg gtg agc aag ggt      1104
   Asn His Glu Tyr Asp Leu Lys Asp Phe Glu Ala Ala Val Ser Lys Gly
           355                 360                 365
   gtg tat ccg gcc gtc tcc tat ctg aag ctg atc gcg gcc cag gat gcc      1152
   Val Tyr Pro Ala Val Ser Tyr Leu Lys Leu Ile Ala Ala Gln Asp Ala
       370                 375                 380
   cat gcc ggc tat tcc gac ccg ctg gac gag cag gaa ggc gtg gtc aag      1200
   His Ala Gly Tyr Ser Asp Pro Leu Asp Glu Gln Glu Gly Val Val Lys
   385                 390                 395                 400
   gtc atc aac ttc ctg atg cgg cag ccg gat tgg aag aac acc gcc atc      1248
   Val Ile Asn Phe Leu Met Arg Gln Pro Asp Trp Lys Asn Thr Ala Ile
                   405                 410                 415
   atc atc acc tat gat gat tcc gat ggc tgg tac gat cat gcc ttc gtg    1296
   Ile Ile Thr Tyr Asp Asp Ser Asp Gly Trp Tyr Asp His Ala Phe Val
               420                 425                 430
   acc ccg acc acc tcc tcc ttc agc ccg atg gac gcg ctg aac ggc aag    1344
   Thr Pro Thr Thr Ser Ser Phe Ser Pro Met Asp Ala Leu Asn Gly Lys
           435                 440                 445
   ggg gtc tgc ggg gcc ggc acc gaa ccg atg ggc ctg gcc ggc aag ccg    1392
   Gly Val Cys Gly Ala Gly Thr Glu Pro Met Gly Leu Ala Gly Lys Pro
       450                 455                 460
   gtg cac ggg ctc tgc ggc ccc ggc acg cgc atc ccc ttc atg gtg atc    1440
   Val His Gly Leu Cys Gly Pro Gly Thr Arg Ile Pro Phe Met Val Ile
   465                 470                 475                 480
   tcg ccc tac gcg aag aaa ggt ttc atc gcc cat acg ctg atc agc cag    1488
   Ser Pro Tyr Ala Lys Lys Gly Phe Ile Ala His Thr Leu Ile Ser Gln
                   485                 490                 495
   gcg tcg gtg gtg aaa ttc atc gag gat aac tgg ctg ggc ggt gcc cgc    1536
   Ala Ser Val Val Lys Phe Ile Glu Asp Asn Trp Leu Gly Gly Ala Arg
               500                 505                 510
   ctg ggc ggc ggc tcc ttc gat gcc aat gcc ggc tcg atc atg gat atg    1584
   Leu Gly Gly Gly Ser Phe Asp Ala Asn Ala Gly Ser Ile Met Asp Met
           515                 520                 525
   ttc gat ttc aag acc gcg ccg aag ggc cgt gat gtg atg ctg ctg gat    1632
   Phe Asp Phe Lys Thr Ala Pro Lys Gly Arg Asp Val Met Leu Leu Asp
       530                 535                 540
   gcc gat acc ggc acc gcc gcg cac gcc gaa taa                        1665
   Ala Asp Thr Gly Thr Ala Ala His Ala Glu
   545                 550                 555
<210> 2
   <211> 554
   <212> PRT
   <213> g. Acidocella
<400> 2
   Met Asn Ile Arg Arg Ala Leu Leu Cys Ala Ser Met Met Val Ser Ser
   1 5 10 15
   Ile Thr Pro Ala Ala Ala Gln Thr Ala Ser Leu Gly Ala Thr Ala Pro
   20 25 30
   Ala Ser Ala Ser Ala Ala Ala Ser Tyr Thr Asp Ala Leu Pro Thr Ala
   35 40 45
   Thr Pro Ile Lys His Leu Val Ile Ile Tyr Gly Glu Asn Val Ser Phe
   50 55 60
   Asp His Tyr Phe Ala Thr Tyr Pro Lys Ala Thr Asn Pro Ala Gly Glu
   65 70 75 80
   Pro Ala Phe His Gly Ser Leu His Gly Gln Lys Ile Asp Asn Leu Val
   85 90 95
   Thr Ala Asn Leu Leu Ser Asn Asn Pro Asn Phe Thr Asn Glu Lys Asn
   100 105 110
   Gly Ala Gly Ala Ala Asn Pro Phe Arg Leu Asp Arg Thr Gln Ala Ala
   115 120 125
   Thr Ala Asp Gln Asn His Ala Tyr Lys Pro Glu Gln Glu Ala Tyr Asp
   130 135 140
   Asn Gly Lys Met Asp Leu Phe Pro Lys Tyr Thr Gly Lys Ala Ser Ala
   145 150 155 160
   Gly Gly Ala Gly Ala Phe Gly Thr Lys Gly Gln Val Met Gly Tyr Phe
   165 170 175
   Asp Gly Asn Thr Val Thr Ala Leu Trp Asn Tyr Ala Gln Gly Tyr Ala
               180                 185                 190
   Met Ser Asp Asn Ala Trp Thr Asp Thr Tyr Gly Pro Ser Thr Pro Gly
           195                 200                 205
   Ala Leu Ala Val Val Ser Gly Gln Thr Asn Gly Ala Val Pro Val Leu
       210                 215                 220
   Gly Thr Ser Pro Asn Leu Asn Pro Asp Gly Gln Gly Gly Phe Thr Asp
   225                 230                 235                 240
   Asp Gly Asp Ile Asp Pro Ala Tyr Asp Val Cys Ser Ser Lys Lys Val
                   245                 250                 255
   Thr Phe Lys Met Glu Gly Gln Asn Ile Gly Asp Leu Leu Asn Ala Lys
               260                 265                 270
   Lys Val Thr Trp Gly Gly Phe Met Gly Gly Phe Asp Leu Gly Leu Thr
           275                 280                 285
   Asn Pro Asn Gly Thr Thr Gly Cys Lys Arg Ser Ser Phe Ala Thr Ala
       290                 295                 300
   Val Asp Ala Pro Thr Ala Asp Tyr Ile Pro His His Asn Trp Phe Gln
   305                 310                 315                 320
   Tyr Tyr Ala Ser Thr Ala Asn Tyr Thr His Ala Arg Pro Ala Ala Leu
                   325                 330                 335
   Asp Asp Ile Gly Tyr Ser Phe Ala Pro Ser Gly Ala Ala Asp Pro Ala
               340                 345                 350
   Asn His Glu Tyr Asp Leu Lys Asp Phe Glu Ala Ala Val Ser Lys Gly
           355                 360                 365
   Val Tyr Pro Ala Val Ser Tyr Leu Lys Leu Ile Ala Ala Gln Asp Ala
       370                 375                 380
   His Ala Gly Tyr Ser Asp Pro Leu Asp Glu Gln Glu Gly Val Val Lys
   385                 390                 395                 400
   Val Ile Asn Phe Leu Met Arg Gln Pro Asp Trp Lys Asn Thr Ala Ile
                   405                 410                 415
   Ile Ile Thr Tyr Asp Asp Ser Asp Gly Trp Tyr Asp His Ala Phe Val
               420                 425                 430
   Thr Pro Thr Thr Ser Ser Phe Ser Pro Met Asp Ala Leu Asn Gly Lys
           435                 440                 445
   Gly Val Cys Gly Ala Gly Thr Glu Pro Met Gly Leu Ala Gly Lys Pro
       450                 455                 460
   Val His Gly Leu Cys Gly Pro Gly Thr Arg Ile Pro Phe Met Val Ile
   465                 470                 475                 480
   Ser Pro Tyr Ala Lys Lys Gly Phe Ile Ala His Thr Leu Ile Ser Gln
                   485                 490                 495
   Ala Ser Val Val Lys Phe Ile Glu Asp Asn Trp Leu Gly Gly Ala Arg
               500                 505                 510
   Leu Gly Gly Gly Ser Phe Asp Ala Asn Ala Gly Ser Ile Met Asp Met
           515                 520                 525
   Phe Asp Phe Lys Thr Ala Pro Lys Gly Arg Asp Val Met Leu Leu Asp
       530                 535                 540
   Ala Asp Thr Gly Thr Ala Ala His Ala Glu
   545                 550
<210> 3
   <211> 20
   <212> ADN
   <213> Séquence artificielle
<220>
   <223> Description de la séquence artificielle: Oligo10
<400> 3
   atdatdatng cngtrttytt 20
<210> 4
   <211> 32
   <212> ADN
   <213> Séquence artificielle
<220>
   <223> Description de la séquence artificielle: Oligo12
<400> 4
   tcrtcrtasg tgatgatgat sgcsgtrtty tt 32
<210> 5
   <211> 23
   <212> ADN
   <213> Séquence artificielle
<220>
   <223> Description de la séquence artificielle: Oligo22
<400> 5
   aaratggarg gncaraayat hgg 23
<210> 6
   <211> 23
   <212> ADN
   <213> Séquence artificielle
<220>
   <223> Description de la séquence artificielle: Oligo23
<400> 6
   atggaaggcc agaayatcgg cga 23
<210> 7
   <211> 23
   <212> ADN
   <213> Séquence artificielle
<220>
   <223> Description de la séquence artificielle: Oligo 25
<400> 7
   atcggcgayc tbctbaaygc caa 23
<210> 8
   <211> 19
   <212> ADN
   <213> Séquence artificielle
<220>
   <223> Description de la séquence artificielle: Oligo29
<400> 8
   ttcatgggtg gcttcgatc 19
<210> 9
   <211> 20
   <212> ADN
   <213> Séquence artificielle
<220>
   <223> Description de la séquence artificielle: Oligo30
<400> 9
   gctgccgcat caggaagttg 20
<210> 10
   <211> 32
   <212> ADN
   <213> Séquence artificielle
<220>
   <223> Description de la séquence artificielle: 5'ORF281
<400> 10
   taacgataac atatgaatat ccgccgggct ct 32
<210> 11
   <211> 33
   <212> ADN
   <213> Séquence artificielle
<220>
   <223> Description de la séquence artificielle: 3'ORF281
<400> 11
   atatctgata agcttttatt cggcgtgcgc ggc 33

## Description

La présente invention concerne de nouvelles phytases d'origine bactérienne, ainsi que leurs procédés de production respectifs. La présente invention concerne plus particulièrement de nouvelles phytases issues de bactéries du genre *Acidocella,* ainsi que les polynucléotides codant pour ces phytases. L'invention concerne également des vecteurs contenant ces polynucléotides, et des organismes hôtes transformés exprimant lesdites phytases dans leurs tissus. L'invention concerne également de nouveaux extraits bactériens comprenant au moins une activité phytase.

En particulier, les extraits bactériens et les phytases de la présente invention sont particulièrement adaptés à leur utilisation dans des compositions alimentaires destinées à l'alimentation animale. Cette adaptation est liée à leurs propriétés, notamment leur activité dans des conditions de température et de pH correspondant aux conditions de préparation desdites compositions ainsi que celles rencontrées dans le système digestif des animaux.

Le phosphore est un élément essentiel à la vie de tous les organismes. En particulier, il est primordial pour les éleveurs d'animaux de ferme de s'assurer que leurs animaux en ingèrent une quantité suffisante pour optimiser leur croissance et leur développement La plupart des animaux de ferme sont nourris avec des compositions alimentaires à base de végétaux. Ces végétaux contiennent de grandes quantités de phosphate qu'ils stockent dans leurs tissus sous forme d'un composé de réserve, l'acide phytique. En moyenne, l'acide phytique contient 50 à 70% du phosphore présent dans les plantes. L'acide phytique est naturellement mobilisé et le phosphate qu'il contient est libéré chez la plupart des animaux de ferme, en particulier les ruminants. Toutefois, l'acide phytique n'est pas métabolisé par les animaux monogastriques tels que le Porc et les volailles. Chez ces animaux, l'acide phytique contenu dans leur ration alimentaire est donc rejeté avec les excréments, et l'éleveur doit compléter ladite ration avec du phosphate inorganique afin que ses animaux ingèrent une quantité suffisante de phosphore. Cette stratégie engendre un surcoût pour l'éleveur et génère une pollution issue du rejet dans l'environnement de l'acide phytique non assimilé. Cette pollution est d'autant plus accrue dans les zones d'élevage intense.

L'acide phytique est également connu pour être un chélateur d'éléments nutritifs importants contenus dans la ration alimentaire, comme par exemple le magnésium, le calcium, le zinc ou le fer. Cette propriété conduit à une diminution de la qualité nutritive de la ration alimentaire donnant à l'acide phytique la propriété d'agent anti-nutritionnel.

Afin de répondre aux différents inconvénients liés à l'absence d'assimilation de l'acide phytique par les animaux monogastriques, il a été envisagé d'introduire une enzyme, la phytase, dans la ration alimentaire de ces animaux d'élevage. La phytase hydrolyse l'acide phytique en libérant de l'inositol et du phosphate inorganique. Des phytases et les gènes codant ces phytases ont été isolés à partir de nombreux organismes. Les phytases ont majoritairement été isolées à partir de champignons (Howson and Davis, 1983, Enzyme Microb. Technol. 5, 377-382; Wyss et al., 1999, Appl. Environ. Microbiol., 65(2), 359-366). Parmi les champignons produisant une phytase, on peut citer les champignons du genre *Aspergillus,* en particulier *A. ficuum* (Ullah et Gibson, 1987, Preparative Biochemistry 17(1), 63-91; Ullah and Dischinger, 1993, Biochem. Biophys. Res. Commun., 192(2), 747-753), *A. terreus* (Mitchell et al., 1997, Microbiology, 143 (Pt 1), 245-252), *A. niger* (Dvorakova et al., 1997, Folia Microbiol (Praha), 42(4), 349-352), *A. fumigatus* (Pasamontes et al., 1997, Appl. Environ. Microbiol., 63(5), 1696-1700), du genre *Penicillium,* en particulier *P. caseicolum,* du genre *Myceliophtora,* en *particulier M. thermophila* (Mitchell et al., 1997, Microbiology, 143 (Pt 1), 245-252), du genre *Talaromyces,* en particulier *T. thermophilus,* du genre *Neurospora,* en particulier *N. crassa* et *N. sitophila,* du genre *Thermomyces,* en particulier *T. lanuginosus* (Berka et al., 1998, Appl Environ Microbiol. 64(11), 4423-4427), ou du genre *Monascus,* en particulier *M. anka.* Des phytases ont également été trouvées chez des bactéries. A titre d'exemple, on peut citer les bactéries des genres *Bacillus,* en particulier *B. subtilis* (Powar et Jagannathan, 1982, J. Bacteriol. 151(3), 102-1108; Shimizu, 1992, Biosci. Biotech. Biocem. 56(8), 1266-1269; Keruvo et al., 1998, Appl. Environ. Microbiol. 64(6), 2079-2085), *Pseudomonas* (Cosgrove, 1970, Austral. J. Biol. Sci. 23, 1207-1220), *Escherichia,* en particulier *E. coli* (Golovan et al., 2000, Can. J. Microbiol. 46, 59-71), *Enterobacter* (Yoon et al., 1996, Enzyme and microbiol. Technol; 18, 449-454), ou *Streptomyces.* Des phytases de levures ont également été isolées (Dvorakova, 1998, Folia Microbiol. 43(4), 323-338), comme celles des levures *Schwaniiomyces occidentalis* et *Saccharomyces cerevisiae.* Enfin, des phytases ont été trouvées dans les plantes, notamment dans le Soja (Ullah et Gibson, 1988, Arch. Biochem. Biophys., 260(2), 514-20), dans le Mais (Maugenest et al., 1997, Biochem J., 322 ( Pt 2), 511-7); Maugenest et al., 1999, Plant Mol. Biol., 39(3), 503-14), ou chez Arabidopsis (Mullaney et Ullah, 1998, Biochem. Biophys. Res. Commun., 251(1), 252-5).

Parmi les propriétés permettant de caractériser les phytases, on trouve la constante de Michaelis (Km) vis-à-vis de l'acide phytique, le pH optimum et la température optimale d'activité, ainsi que la stabilité de cette activité à des pH et des températures données. Des données relatives à la structure des phytases peuvent également être utiles, comme le poids moléculaire (PM), le point isoélectrique (pI), ou la séquence peptidique. Pour être utilisables en alimentation animale, les phytases doivent posséder des propriétés compatibles avec les traitements que subissent les aliments destinés à cette alimentation. En particulier, l'activité des phytases utilisées doit se maintenir et si possible être optimale aux conditions de température et de pH des procédés de préparation de ces aliments ainsi qu'à celles présentes dans le tractus digestif des animaux ingérant ces aliments. Ces contraintes conduisent principalement à rechercher des phytases dont l'activité résiste à des conditions de température élevées, telles que celles utilisées dans les procédés de préparation des compositions alimentaires, et à des conditions de pH acides, telles que celles présentes dans le tractus digestif des animaux d'élevage.

Afin de répondre à ces critères, des phytases ont été recherchées dans des organismes, en particulier des micro-organismes, se développant dans des milieux dont les conditions de température et de pH correspondent à ces critères. Cette stratégie a permis d'isoler des phytases résistantes à des températures élevées, telles que par exemple celles décrites dans les demandes de brevets WO 97/35016 ou EP 0 684 313, mais aussi des phytases possédant un faible Km, telles que par exemple celles décrites dans la demande de brevet EP 0 960 934. Une autre stratégie a consisté à modifier artificiellement la séquence de phytases connues par mutagenèse dirigée afin de lui conférer des propriétés intéressantes. Cette stratégie à notamment été décrite dans les demandes de brevets WO 99/48380, EP 0 897 985 et EP 0 897 010.

Seules quelques phytases actives à un faible pH optimal ont été identifiées. De telles phytases ont notamment été identifiées chez les champignons, comme la phytase de *Penicillim caseicolum* décrite dans la demande de brevet JP 7067635 et celle de *Monascus anka* décrite dans la demande de brevet WO 98/13480, mais aussi chez les levures, comme la phytase de *Schwaniiomyces occidentalis* décrite dans la demande de brevet EP 699 762. Toutefois, jusqu'à présent, aucune bactérie n'a conduit à l'isolement et a l'identification d'une phytase possédant un optimum de pH faible, en particulier un pH optimum inférieur à 4.

### Description des Figures

Figure 1: Activité de la phytase d'*Acidocella aminolytica* ATCC 51361 en fonction de la température. La valeur 100% de l'activité relative correspond à l'activité optimale de la phytase pour une température donnée.
Figure 2: Activité de la phytase d'*Acidocella aminolytica* ATCC 51361 en fonction du pH. La valeur 100% de l'activité relative correspond à l'activité optimale de la phytase pour un pH donné.
Figure 3: Activité de la phytase d'*Acidocella facilis* ATCC 35904 en fonction de la température. La valeur 100% de l'activité relative correspond à l'activité optimale de la phytase pour une température donnée.
Figure 4: Activité de la phytase d'*Acidocella facilis* ATCC 35904 en fonction du pH. La valeur 100% de l'activité relative correspond à l'activité optimale de la phytase pour un pH donné.
Figure 5: Activité de la phytase contenue dans la fraction purifiée à l'exemple 5.5 et codée par l'ORF281 en fonction de la température. La valeur 100% de l'activité relative correspond à l'activité optimale de la phytase pour une température donnée.

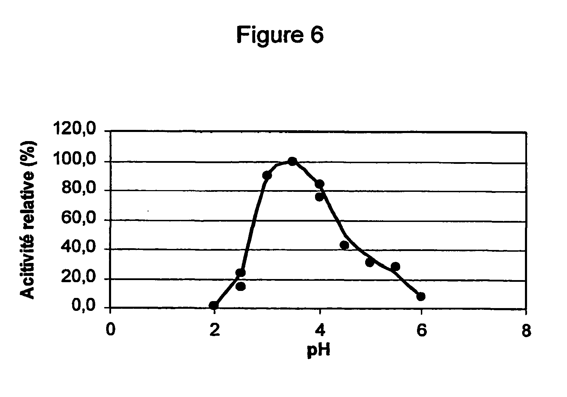

Figure 6: Activité de la phytase contenue dans la fraction purifiée à l'exemple 5.5 et codée par l'ORF281 en fonction du pH. La valeur 100% de l'activité relative correspond à l'activité optimale de la phytase pour un pH donné.

### Description

La présente invention concerne des polynucléotides isolés codant pour une phytase décrite par l'identificateur de séquence SEQ ID NO:2. Cette phytase et les polynucléotides qui la codent sont également **caractérisés en ce qu**'ils proviennent d'une souche bactérienne du genre *Acidocella.*

Selon la présente invention, on entend par "polynucléotide" une molécule d'acide nucléique composée d'une séquence de bases, naturelle ou artificielle, pouvant être de type ADN ou ARN, de préférence de type ADN, notamment double brin. Lorsque ledit polynucléotide est naturel, il est bien entendu que l'invention ne couvre pas ce polynucléotide dans son environnement naturel, mais le même polynucléotide isolé et purifié du génome de l'organisme vivant dans lequel il se trouve à l'état naturel. Ce polynucléotide peut être obtenu de manière directe, par extraction et purification, ou de manière indirecte par copie. Toutefois, la présente invention comprend ledit polynucléotide lorsqu'il est intégré de manière artificielle dans le génome d'un organisme vivant autre que celui dans lequel il existe à l'état naturel, ou lorsqu'il est réintroduit artificiellement dans l'organisme vivant duquel il provient, en un ou plusieurs exemplaires dans le génome de cet organisme. Lorsque ce polynucléotide est une sonde, il est généralement simple brin.

L'invention comprend donc les polynucléotides codant pour la séquence peptidique de la phytase décrite par l'identificateur de séquence SEQ ID NO:2. Il est bien connu de l'homme du métier que cette définition inclut tous les polynucléotides qui, bien que comprenant des séquences nucléotidiques différentes comme résultat de la dégénérescence du code génétique, codent pour une même séquence d'acides aminés, donc une même phytase, laquelle est représentée par l'identificateur de séquences SEQ ID NO: 2.

La présente invention comprend également des polynucléotides homologues des polynucléotides précédemment décrits, lesdits polynucléotides homologues codant pour des phytases homologues de la phytase représentée par l'identificateur de séquence SEQ ID NO:2. Par "homologue", on entend selon l'invention des polynucléotides codant des phytases et dont les séquences présentent des modifications par rapport aux polynucléotides codant la phytase représentée par l'identificateur de séquence SEQ ID NO:2. Les polynucléotides homologues sont caractérisés par un degré d'identité avec les polynucléotides codant la phytase représentée par l'identificateur de séquence SEQ ID NO:2. Le degré d'identité entre deux polynucléotides homologues est obtenu par comparaison de leurs séquences et est généralement exprimé par un pourcentage de nucléotides identiques entre ces séquences. Ce degré d'identité est mesuré sur une longueur de séquence donnée, la plus courte des séquences comparées déterminant la taille de séquence sur laquelle le degré d'identité des séquences homologues est mesuré. L'invention couvre donc des polynucléotides présentant une ou plusieurs modifications de séquence par rapport aux polynucléotides codant la phytase représentée par l'identificateur de séquence SEQ ID NO:2, et codant pour des phytases dont les propriétés sont équivalentes à celles de la phytase décrite par l'identificateur de séquence SEQ ID NO:2.

Par "phytases équivalentes" ou "phytases aux propriétés équivalentes", on entend essentiellement selon l'invention des protéines possédant une activité phytase, indépendamment de leurs propriétés intrinsèques telles que le Km, le pH optimal d'activité ou la température optimale d'activité. Le niveau d'activité phytase peut être mesuré par toute méthode permettant de caractériser une activité phytase. Par phytase, on entend une enzyme dont l'activité catalytique consiste à hydrolyser l'acide phytique pour libérer de l'inositol et du phosphate inorganique. Toutefois, la plupart des phytases ne réalisant pas une hydrolyse complète de l'acide phytique (comportant 6 phosphates), l'activité catalytique d'une phytase selon l'invention peut conduire à la libération de phosphate inorganique et d'esters de phosphate-myoinositol, lesdits esters pouvant être, en fonction de la capacité d'hydrolyse de la phytase, des esters de myoinositol mono-, di-, tri, tétra- ou penta-phosphate. A titre d'exemple, l'activité phytase peut être mesurée selon la méthode de Shimizu (1992, Biosci. Biotech. Biochem. 56(8), 1266-1269), en particulier telle que décrite dans l'exemple 2. Toutefois, toute méthode permettant de caractériser une activité phytase, soit par la mesure de la diminution de la quantité de substrat, soit par la mesure de l'accumulation des produits issus de la réaction enzymatique, peut être utilisée pour mesurer l'activité phytase. En particulier, des méthodes semblables, utilisant par exemple un autre substrat ou d'autres réactifs, permettent également de mesurer ladite activité phytase.

Les modifications de séquences présentes dans les polynucléotides homologues peuvent être des additions, délétions, ou substitutions de nucléotides qui peuvent être naturelles ou obtenues par les techniques usuelles de mutagenèse. Il est connu que de tels polynucléotides homologues, codant pour des protéines de fonctions équivalentes, existent naturellement dans les génomes d'espèces vivantes différentes, et même dans les génomes de races, variétés ou souches différentes. Par conséquent, il est donc aisé pour un homme du métier, à partir de l'enseignement des polynucléotides codant pour la séquence peptidique représentée par l'identificateur de séquence SEQ ID NO:2 selon l'invention, d'isoler de tels polynucléotides homologues en utilisant des techniques bien connues d'hybridation moléculaire (Sambrook et al., 1989, Molecular Cloning : A Laboratory Manual, 2nd edition, Nolan C. ed., New York: Cold Spring Harbor Laboratory Press).

L'hybridation moléculaire est une réaction d'appariement qui s'effectue entre des brins complémentaires de polynucléotides présentant un certain degré d'identité entre leurs séquences nucléotidiques. L'hybridation permet donc, à partir des polynucléotides codant la phytase représentée par l'identificateur de séquence SEQ ID NO:2, d'identifier des polynucléotides homologues de ceux-ci dans le génome d'organismes vivants autres que celui dont ils sont issus, par exemple d'autres bactéries, en particulier d'autres souches d'*Acidocella,* et codant pour des phytases aux propriétés équivalentes à la phytase représentée par l'identificateur de séquence SEQ ID NO:2. Plus l'identité de séquence entre des polynucléotides est forte, plus l'hybridation entre lesdits polynucléotides est possible et aisée, et plus la probabilité que ces polynucléotides codent pour des protéines aux propriétés équivalentes est forte. Les méthodes permettant d'hybrider des polynucléotides sont largement décrites dans la littérature (Sambrook et aL, 1989, Molecular Cloning : A Laboratory Manual, 2nd edition, Nolan C. ed., New York: Cold Spring Harbor Laboratory Press) et bien connues de l'homme du métier. Elles sont, par exemple, basées sur le criblage d'une banque génomique ou de cDNA créée à partir d'un organisme vivant ou d'un tissu de cet organisme. Le criblage s'effectue à l'aide d'une sonde constituée d'un polynucléotide connu ou un fragment de celui-ci, afin d'identifier dans ces banques les polynucléotides homologues à ladite sonde qui vont s'hybrider à celle-ci. Selon l'invention, la sonde est constituée d'un polynucléotide codant la phytase représentée par l'identificateur de séquence SEQ ID NO:2, ou un fragment de ceux-ci. Afin d'identifier les polynucléotides sur lesquels la sonde s'hybride, celle-ci est marquée, par exemple avec des éléments radioactifs, comme le ³²P. Des marqueurs non-radioactifs commercialisés et bien connus de l'homme du métier peuvent également être utilisés.

La présente invention comprend donc également des polynucléotides capables de s'hybrider de manière sélective à un des polynucléotides codant la phytase représentée par l'identificateur de séquence SEQ ID NO:2. Il est entendu que ces polynucléotides ne font partie de la présente invention que s'ils codent pour une phytase équivalente à celle représentée par l'identificateur de séquence SEQ ID NO:2. Par "polynucléotides capables de s'hybrider de manière sélective", on entend donc selon l'invention les polynucléotides qui, par une des méthodes usuelles d'hybridation moléculaire (Sambrook et al., 1989, Molecular Cloning : A Laboratory Manual, 2nd edition, Nolan C. ed., New York: Cold Spring Harbor Laboratory Press), s'hybrident avec une sonde marquée constituée par un des polynucléotides codant la phytase représentée par l'identificateur de séquence SEQ ID NO:2, ou un fragment de ceux-ci, à un niveau supérieur à l'hybridation non spécifique de ladite sonde avec d'autres polynucléotides peu homologues, notamment d'autres ADNc si les polynucléotides sondés sont issus d'une banque d'ADNc. Le niveau d'hybridation est mesuré grâce au signal produit par le marqueur de la sonde. Le niveau du signal généré par l'interaction entre les polynucléotides capables de s'hybrider de manière sélective et la sonde est généralement 10 fois, de préférence 100 fois plus intense que celui généré par l'interaction de la sonde avec les autres polynucléotides générant un "bruit de fond". L'hybridation sélective est obtenue en employant des conditions d'hybridation et de lavage stringentes ou très stringentes (par exemple hybridation avec un tampon contenant au moins 5 x SSC et 1% SDS à environ 50°C-60°C, et lavages successifs avec 0,1% SDS et diminution progressive de la concentration en SSC de 2xSSC à 0,4xSSC ainsi qu'augmentation de la température de 20°C à 50°C). L'homme du métier saura adapter les conditions d'hybridation, c'est-à-dire essentiellement la température et la concentration saline des tampons employés pour l'étape d'hybridation et l'étape de lavage. Ces conditions doivent notamment être adaptées en fonction de la taille de la sonde utilisée et du degré d'identité des polynucléotides présents dans la banque criblée avec cette sonde. L'adaptation des conditions d'hybridation est nécessaire afin d'optimiser le signal généré par les séquences homologues s'hybridant, tout en minimisant le bruit de fond.

Les polynucléotides capables de s'hybrider de manière sélective aux polynucléotides selon l'invention peuvent être isolés à partir de banques génomiques ou de banques d'ADNc produites, par exemple, à partir de souches bactériennes, en particulier de souches du genre *Acidocella.* L'isolement de tels polynucléotides peut se faire par les techniques standards d'hybridation (Sambrook et al., 1989, Molecular Cloning : A Laboratory Manual, 2nd edition, Nolan C. ed., New York: Cold Spring Harbor Laboratory Press), en utilisant comme sonde un polynucléotide codant la phytase représentée par l'identificateur de séquence SEQ ID NO:2, un fragment de ces polynucléotides, ou un polynucléotides complémentaire de ceux-ci. Lorsqu'un polynucléotide a été isolé par ces techniques, il est nécessaire d'en déterminer la séquence et d'identifier les propriétés de la protéine codée par ce polynucléotide, en particulier de vérifier que cette protéine est une phytase équivalente à la phytase décrite par l'identificateur de séquence SEQ ID NO:2.

Les techniques d'hybridation ci-dessus mentionnées permettent donc d'isoler des polynucléotides homologues des polynucléotides codant pour la phytase décrite par l'identificateur de séquence SEQ ID NO:2. De tels polynucléotides, et les phytases qu'ils codent, sont aisément identifiables par un homme du métier du domaine des biotechnologies maîtrisant les techniques standards de biologie moléculaire. L'invention comprend donc des polynucléotides homologues des polynucléotides codant la phytase décrite par l'identificateur de séquence SEQ ID NO:2. Le degré d'identité des polynucléotides homologues sera d'au moins 70 % par rapport aux polynucléotides codant la phytase représentée par l'identificateur de séquence SEQ ID NO:2, de préférence d'au moins 80%, plus préférentiellement d'au moins 90 %, et de manière préférée d'au moins 95%. Les méthodes de mesure et d'identification du degré d'identité entre des séquences sont bien connues de l'homme du métier. On peut employer par exemple les programmes PILEUP, BLAST (notamment Altschul et al., 1993, J. Mol. Evol. 36 :290-300 ; Altschul et al., 1990, J. Mol. Biol. 215 :403-10), ou BestFit (Wisconsin Sequence Analysis Package, Version 8 for Unix, Genetics Computer Group, University Research Park, 575 Science Drive, Madison, WI 53711, utilisant l'algorithme de Smith et Waterman décrit dans Applied Mathematics, 1981, No.2, 482-489).

De tels polynucléotides homologues peuvent également être obtenus de manière artificielle par les techniques classiques de mutagenèse.

Un polynucléotide préféré codant la phytase représentée par l'identificateur de séquence SEQ ID NO:2 est représenté par l'identificateur de séquence SEQ ID NO:1.

Selon un mode particulier de réalisation de l'invention, les polynucléotides codant une phytase décrits ci-dessus proviennent d'une bactérie du genre *Acidocella.*

De manière avantageuse, les polynucléotides selon l'invention codent une phytase dont le pH optimal d'activité est inférieur ou égal à 4. Par pH optimal d'activité, on entend la valeur de pH à laquelle l'activité de la phytase selon l'invention est maximale, cette activité étant mesurée par la méthode ci-dessus mentionnée et correspondant à la quantité d'acide phytique dégradé et de phosphate inorganique produit.

Selon un mode particulier de réalisation de l'invention, les polynucléotides selon l'invention codent une phytase possédant les propriétés suivantes:
(a) température optimale = 70°C
(b) pH optimal = 4

Selon un autre mode particulier de réalisation de l'invention, les polynucléotides selon l'invention codent une phytase possédant les propriétés suivantes:
(a) température optimale = 60°C
(b) pH optimal = 3,5

De préférence, les polynucléotides selon l'invention codent une phytase provenant d'une souche bactérienne de l'espèce *Acidocella aminolytica,* en particulier la souche *A. aninolytica* ATCC 51361, ou d'une souche bactérienne de l'espèce *Acidocella facilis,* en particulier la souche *A. facilis* ATCC 35904.

La présente invention décrit également des fragments des polynucléotides décrits ci-dessus. Le terme "fragment" désigne notamment un fragment d'au moins 20 nucléotides, en particulier d'au moins 50 nucléotides, et de préférence d'au moins 100 nucléotides. De tels fragments sont généralement désignés oligonucléotides. Ils peuvent servir de sondes d'hybridation pour identifier des polynucléotides homologues, ou d'amorces pour identifier et amplifier de tels polynucléotides homologues par la technique de PCR ("Polymerase Chain Reaction" telle que décrite dans Ausubel et al., (1987) Current Protocols in Molecular Biology, edit, John Wiley & Sons, Section 6.3-6.4.

Le terme fragment désigne également des fragments des polynucléotides selon l'invention codant un fragment de la phytase représentée par l'identificateur de séquence SEQ ID NO:2, ou un fragment d'une phytase homologue ou équivalente de la phytase représentée par l'identificateur de séquence SEQ ID NO:2.

La présente invention concerne également des polynucléotides comprenant au moins un des polynucléotides tels que décrits précédemment.

Tous les polynucléotides décrits ci-dessus codent soit la phytase représentée par l'identificateur de séquence SEQ ID NO:2, soit une phytase homologue, soit un fragment actif de ces phytases. En conséquence, l'invention s'étend donc à toutes les phytases codées par l'ensemble de ces polynucléotides. Cette définition inclue donc la phytase représentée par l'identificateur de séquence SEQ ID NO:2, les phytases homologues de cette phytase, et les fragments actifs de ces phytases.

Selon un mode préféré de réalisation de l'invention, la phytase est une protéine comprenant au moins la séquence peptidique décrite par l'identificateur de séquence SEQ ID NO:2. De manière préférée, la phytase représentée par la séquence peptidique décrite par l'identificateur de séquence SEQ ID NO:2 hydrolyse 5 des 6 phosphates présents sur une molécule d'acide phytique.

L'invention comprend donc également les phytases homologues de la phytase représentée par l'identificateur de séquence SEQ ID NO:2. Par "phytases homologues", on entend selon l'invention les phytases dont les séquences présentent des modifications par rapport à la phytase représentée par l'identificateur de séquence SEQ ID NO:2. À l'instar des polynucléotides homologues, les phytases homologues sont des phytases dont les séquences peptidiques présentent un certain degré d'identité, lequel degré d'identité est généralement exprimé par un pourcentage d'acides aminés identiques. L'invention couvre donc des phytases présentant une ou plusieurs modifications de séquence par rapport à la phytase représentée par l'identificateur de séquence SEQ ID NO:2, et dont les propriétés sont équivalentes à celles de la phytase décrite par l'identificateur de séquence SEQ ID NO:2. Ces modifications peuvent être des additions, délétions, ou substitutions d'acides aminés qui peuvent être naturelles ou obtenues par les techniques usuelles de mutagenèse. Le degré d'identité des phytases homologues sera d'au moins 70 % par rapport à la phytase représentée par l'identificateur de séquence SEQ ID NO:2, de préférence d'au moins 80%, plus préférentiellement d'au moins 90 %, et de manière préférée d'au moins 95%. Les méthodes de mesure et d'identification du degré d'identité entre des séquences sont bien connues de l'homme du métier. On peut employer par exemple les programmes PILEUP, BLAST (notamment Altschul et al., 1993, J. Mol. Evol. 36 :290-300 ; Altschul et al., 1990, J. Mol. Biol. 215 :403-10), ou BestFit (Wisconsin Sequence Analysis Package, Version 8 for Unix, Genetics Computer Group, University Research Park, 575 Science Drive, Madison, WI 53711, utilisant l'algorithme de Smith et Waterman décrit dans Applied Mathematics, 1981, No.2, 482-489).

Selon un mode particulier de réalisation de l'invention, la phytase selon l'invention provient d'une bactérie du genre *Acidocella.*

De manière avantageuse, la phytase selon l'invention a un pH optimal d'activité inférieur ou égal à 4. Par pH optimal d'activité, on entend la valeur de pH à laquelle l'activité de la phytase selon l'invention est maximale, cette activité étant mesurée par la méthode ci-dessus mentionnée et correspondant à la quantité d'acide phytique dégradé et de phosphate inorganique produit.

Selon un mode particulier de réalisation de l'invention, la phytase possède les propriétés suivantes:
(a) température optimale = 70°C
(b) pH optimal = 4

Selon un autre mode particulier de réalisation de l'invention, la phytase possède les propriétés suivantes:
(a) température optimale = 60°C
(b) pH optimal = 3,5

Selon un autre mode particulier de réalisation de l'invention" la phytase selon l'invention provient d'une souche bactérienne de l'espèce *Acidocella aminolytica,* en particulier la souche *A. aminolytica* ATCC 51361, ou d'une souche bactérienne de l'espèce *Acidocella facilis,* en particulier la souche *A. facilis* ATCC 35904.

Sont également décrits fragments de la phytase représentée par l'identificateur de séquence SEQ ID NO:2 et aux fragments des phytases homologues. Par fragment, on entend essentiellement un fragment biologiquement actif, c'est-à-dire un fragment possédant une activité phytase équivalente à celle de la phytase complète telle que mesurée par le test décrit dans l'exemple 2, ou un test semblable.

La présente invention concerne également un gène chimère comprenant au moins, liés entre eux de manière opérationnelle, un promoteur fonctionnel dans un organisme hôte, un polynucléotide codant une phytase selon l'invention, et un élément terminateur fonctionnel dans ce même organisme hôte. Les différents éléments qu'un gène chimère peut contenir sont, d'une part, des éléments régulateurs de la transcription, de la traduction et de la maturation des protéines, tels qu'un promoteur, une séquence codant pour un peptide signal ou un peptide de transit, ou un élément terminateur constituant un signal de polyadénylation, et d'autre part un polynucléotide codant pour une protéine. L'expression "liés entre eux de manière opérationnelle" signifie que lesdits éléments du gène chimère sont liés entre eux de manière à ce que le fonctionnement d'un de ces éléments est affecté par celui d'un autre. A titre d'exemple, un promoteur est lié de manière opérationnelle à une séquence codante lorsqu'il est capable d'affecter l'expression de ladite séquence codante. La construction du gène chimère selon l'invention et l'assemblage de ses différents éléments est réalisable par l'emploi de techniques bien connues de l'homme du métier, notamment celles décrites dans Sambrook et al. (1989, Molecular Cloning : A Laboratory Manual, Nolan C. ed., New York: Cold Spring Harbor Laboratory Press). Le choix des éléments régulateurs constituant le gène chimère est essentiellement fonction de l'organisme hôte dans lequel ils doivent fonctionner, et l'homme du métier est capable de sélectionner des éléments régulateurs fonctionnels dans un organisme hôte donné. Par "fonctionnels", on entend capables de fonctionner dans un organisme hôte donné.

Les promoteurs que peut contenir le gène chimère selon l'invention sont soit constitutifs, soit inductibles. A titre d'exemple, les promoteurs utilisés pour l'expression dans des bactéries pourront être choisis parmi les promoteurs cités ci-dessous. Pour l'expression chez *Escherichia coli,* on peut citer les promoteurs *lac, trp, lpp, phoA, recA, araBAD, proU, cst-1, tetA, cadA, nar, tac, trc, lpp-lac,* P*syn, cspA,* P*L,* P*L-9G-50,* P*R-PL, T7*, λP*L*-PT7, T3-*lac,* T5-*lac,* T4 gene 32, *nprM-lac*, VHb, Proteine A (Makrides, 1996, Microbiol. Rev. 60:512-538; Current Opinions in Biotechnology, 1996, 7; Weickert et al., 1996, Current Opinions in Biotechnology 7 : 494-499) ou encore le promoteur *Pₜᵣₚ* (WO 99/64607). Pour l'expression chez les bactéries Gram positives comme les Corynébactéries ou *Streptomyces*, on peut citer les promoteurs P*_{tipA}* (Holmes et al., 1993, EMBO J. 12:3183-3191) ou PS1, PS2 (FR91/09870) ou ceux décrit dans la demande EP0629699A2. Pour l'expression chez les levures et champignons, on peut citer les promoteurs P_{LAC4} de *K*. *lactis* (Van den Berg et al., 1990, Bio/Technology 8:135-139) ou P*_{pgk}* de *K*. *lactis* (Demande de brevet FR 91/05294), *tef1* ou *cbh1* de *Trichoderma* (WO94/04673), *his, csl, apf* de *Penicillium* (WO 00/68401), *gla* d'*Aspergillus* (Gwynne et al., 1987, Bio/Technology 5: 713-719).

Le gène chimère peut également comprendre une séquence d'adressage subcellulaire, codant un peptide signal ou un peptide de transit. Une telle séquence, située en amont ou en aval du polynucléotide codant pour la phytase, permet de diriger ladite phytase de manière spécifique dans un compartiment cellulaire de l'organisme hôte, ou de diriger sa sécrétion dans l'espace extracellulaire. Par exemple, le gène chimère peut comprendre une séquence codant un peptide signal ou un peptide de transit permettant de diriger la phytase vers un compartiment particulier du cytoplasme comme les mitochondries, les plastes, le réticulum endoplasmique ou les vacuoles. De manière préférée, la séquence d'adressage code pour un peptide signal adressant la phytase dans l'apoplaste ou la matrice extracellulaire.

Selon un mode de réalisation, le peptide de transit peut être un signal d'adressage chloroplastique, vacuolaire ou mitochondrial, lequel est ensuite clivé dans les chloroplastes, les vacuoles ou les mitochondries. De tels peptides sont largement décrits dans la littérature: Neuhaus and Rodgers, 1998, Plant Molecular Biology 38: 127-144; peptide de transit de l'EPSPS décrit dans le brevet USP 5188642; peptide de transit de la petite sous-unité de la ribulose-biscarboxylase/oxyge,ase (EP 189707).

Selon un autre mode de réalisation, le peptide de transit peut être constitué par un peptide signal d'adressage dans le périplasme bactérien comme ceux des gènes *pac* (Schumacher et al., 1986, Nucl. Acids. Res. 14:5713-5727), *ompA* (Bowden et Georgiou, 1990, J. Biol. Chem. 265: 16761-16766), *phoA* (Chang et al., 1986, Gene 44: 121-124), ou par un peptide d'ancrage à la surface de la bactérie comme ceux des gènes PS1 et PS2 (FR91/09870).

Les peptides de transit peuvent être soit simples, soit doubles. Les peptides de transit doubles sont éventuellement séparés par une séquence intermédiaire. A titre d'exemple de peptide de transit chloroplastique, on peut citer un peptide de transit double comprenant, dans le sens de la transcription, une séquence codant pour un peptide de transit d'un gène végétal codant pour une enzyme à localisation plastidiale, une partie de séquence de la partie mature N-terminale d'un gène végétal codant pour une enzyme à localisation plastidiale, puis une séquence codant pour un second peptide de transit d'un gène végétal codant pour une enzyme à localisation plastidiale. De tels peptides de transit chloroplastiques doubles sont par exemple décrits dans la demande de brevet EP 0 508 909.

Selon un mode de réalisation, le peptide de transit peut se composer de différents éléments permettant d'augmenter le taux de protéine d'intérêt sécrétée dans le milieu. On peut ainsi citer l'association d'une protéine porteuse et d'un site de clivage protéolytique fusionné avec la protéine d'intérêt (USP 6130063).

Selon l'invention, le gène chimère peut également comprendre d'autres séquences de régulation, qui sont situées entre le promoteur et la séquence codante, telles que des activateurs de transcription (*enhancer*).

La présente invention concerne également un vecteur de clonage et/ou d'expression comprenant un gène chimère selon l'invention. Le vecteur selon l'invention est utile pour transformer un organisme hôte et exprimer dans celui-ci une phytase. Ce vecteur peut être un plasmide, un cosmide, un bactériophage ou un virus. De manière préférentielle, le vecteur de transformation selon l'invention est un plasmide. De manière générale, les principales qualités de ce vecteur doivent être une capacité à se maintenir et à s'autorépliquer dans les cellules de l'organisme hôte, notamment grâce à la présence d'une origine de réplication, et à y exprimer une phytase. Dans le but d'une transformation stable d'un organisme hôte, le vecteur peut aussi s'intégrer dans le génome. Sa composition peut alors se limiter aux éléments nécessaires à la synthèse de la phytase chez ces hôtes. Le choix d'un tel vecteur ainsi que les techniques d'insertion dans celui-ci du gène chimère selon l'invention sont largement décrits dans Sambrook et al. (1989, Molecular Cloning: A Laboratory Manual, Nolan C. ed., New York: Cold Spring Harbor Laboratory Press) et font partie des connaissances générales de l'homme du métier. De manière avantageuse, le vecteur utilisé dans la présente invention contient également, en plus du gène chimère selon l'invention, un gène chimère codant un marqueur de sélection. Ce marqueur de sélection permet de sélectionner les organismes hôtes effectivement transformés, c'est-à-dire ceux ayant incorporé le vecteur. Selon un mode particulier de réalisation de l'invention, l'organisme hôte à transformer est une plante ou un champignon. Parmi les marqueurs de sélection utilisables, on peut citer des marqueurs contenant des gènes de résistance aux antibiotiques ou aux fongicides tels que, par exemple, le gène de l'hygromycine phosphotransférase (Gritz et al., 1983, Gene 25:179-188; Punt et al., 1987; Gene, 56: 117-24), celui de la streptothricine acetyltransferase, celui codant pour un polypeptide conférant une résistance à la phléomycine, celui de la beta-tubuline mutée conférant la résistance au benomyl (Gold et al., 1994, Gene 142: 225-30), ou celui de la bialaphos-acetyltransferase (Avalos et al., 1989, Curr Genet, 16:369-72). D'autres marqueurs peuvent être des gènes complémentant une auxotrophie comme les gènes pyrA, pyrB, pyrG, pyr4 (Buxton et Radford, 1983, Mol. Gen. Genet. 190 : 403-405), arg4, argB (Berse et al., 1983, Gene 25 : 109-117), trpC (Goosen et al., 1989, Mol Gen Genet, 219:282-88), le gène de la molybdopterine synthase (Appleyard et al., 1998, J Biol Chem 273: 14869-76 ; Unkles et al., 1999; J Biol Chem, 274:19286-93), ou celui de l'acétamidase (Beri and Turner, 1987, Curr Genet, 11:639-41). Une autre catégorie de marqueurs de sélection est constituée par des gènes de tolérance aux herbicides tel que le gène *bar* (White et al., NAR 18:1062, 1990) pour la tolérance au bialaphos, le gène EPSPS (US 5,188,642) pour la tolérance au glyphosate, le gène HPPD (WO 96/38567) pour la tolérance aux isoxazoles, ou encore le gène de la glyphosate oxydoreductase (US5463175). On peut également citer des gènes codant pour des enzymes facilement identifiables comme l'enzyme GUS, des gènes codant pour des pigments ou des enzymes régulant la production de pigments dans les cellules transformées. De tels gènes marqueurs de sélection sont notamment décrits dans les demandes de brevet WO 91/02071, WO 95/06128, WO 96/38567, et WO 97/04103.

La présente invention concerne également des organismes hôte transformés, contenant au moins un gène chimère selon l'invention soit intégré dans leur génome, soit porté sur un élément génétique extra-chromosomique, par exemple un plasmide. Par organisme hôte, on entend tout organisme mono ou pluricellulaire, inférieur ou supérieur, dans lequel le gène chimère selon l'invention peut être introduit, pour la production d'une phytase selon l'invention. De manière préférée, l'organisme hôte est un microorganisme, en particulier un champignon, par exemple des genres *Penicillium, Aspergillus, Chrysosporium,* ou *Trichoderma,* une bactérie, par exemple du genre *Escherichia,* en particulier *E. coli,* du genre *Corynebacterium* ou du genre *Streptomyces,* une levure, en particulier des genres *Saccharomyces, Kluyveromyces,* ou *Pichia,* un baculovirus, ou des cellules végétales. L'organisme hôte peut également être une plante, ou une partie de plante.

On entend par "organisme hôte transformé", un organisme hôte qui a incorporé dans son génome, ou dans un élément génétique extra-chromosomique, par exemple un plasmide, au moins un gène chimère selon l'invention, et produit en conséquence une phytase dans ses tissus, ou dans un milieu de culture. Pour obtenir les organismes hôtes selon l'invention, l'homme du métier peut utiliser une des nombreuses méthodes de transformation connues.

Une de ces méthodes consiste à mettre les cellules ou tissus des organismes hôtes à transformer en présence de polyéthylène glycol (PEG) et des vecteurs de l'invention (Chang and Cohen, 1979, Mol. Gen. Genet 168(1), 111-115; Mercenier and Chassy, 1988, Biochimie 70(4), 503-517). L'électroporation est une autre méthode qui consiste à soumettre les cellules ou tissus à transformer et les vecteurs de l'invention à un champ électrique (Andreason and Evans, 1988, Biotechniques 6(7), 650-660; Shigekawa and Dower, 1989, Aust. J. Biotechnol. 3(1), 56-62). Une autre méthode consiste à directement injecter les vecteurs dans les cellules ou les tissus par micro-injection (Gordon and Ruddle, 1985, Gene 33(2), 121-136). De manière avantageuse, la méthode dite de "biolistique" pourra être utilisée. Elle consiste à bombarder des cellules ou des tissus avec des particules sur lesquelles sont adsorbés les vecteurs de l'invention (Bruce et al., 1989, Proc. Natl. Acad. Sci. USA 86(24), 9692-9696; Klein et al., 1992, Biotechnology 10(3), 286-291; US Patent No. 4,945,050).

Plusieurs méthodes de transformation de bactéries sont décrites dans la littérature pour *Escherichia coli* et d'autres bactéries Gram-négatives (Ausubel et al., 1995, Current Protocols in Molecular Biology, John Willey and Sons, New York; Inoue et al., 1990, Gene 96: 23-28; Chung et al., 1989, Proc. Natl. Acad. Sci. USA 86: 2172-2175). La conjugaison peut également être utilisée (Cameron et al., 1989, J. Bacteriol., 171: 547-557). Pour les bactéries Gram-positives l'électroporation peut être utilisée ainsi que la transformation de protoplastes, en particulier pour les bactéries du genre *Streptomyces* (Bonamy et al., 1990, FEMS Microbio. Lett 66: 263-270; Hopwood et al., 1985, Genetic Manipulation of Streptomyces: A Laboratory Manual. John Innes Foundation, Norwich).

Plusieurs méthodes de transformation de champignons sont également décrites dans la littérature (Talbot, 2001, Molecular and cellular biology of filamentous fungi, Oxford University Press, New York). La transformation de protoplastes avec du PEG est décrite pour *Aspergillus* dans EP 0260762, et une adaptation de cette méthode à l'espèce *Penicillium funiculosum* dans WO 00/36120. On connaît également la transformation par intégration assistée d'enzyme de restriction ou REMI (Sanchez et al., 1998, Mol. Gen. Genet. 258 ; 89-94), ainsi que la transformation de protoplastes à l'aide à l'aide de bactéries du genre *Agrobacterium* (de Groot et al., 1998, Nature Biotechnology 16: 839-842). Des techniques de transformation de levures sont également décrites dans la littérature, notamment dans Ausubel et al. (1995, Current Protocols in Molecular Biology, John Willey and Sons, New York) et Van den Berg et al. (1990, Bio/Technology 8: 135-139).

Dans le cas particulier où l'organisme hôte à transformer est d'origine végétale, la transformation de cellules ou tissus végétaux peut se faire préférentiellement à l'aide de bactéries du genre *Agrobacterium,* de préférence par infection des cellules ou tissus desdites plantes par *A. tumefaciens* (Knopf, 1979, Subcell. Biochem. 6, 143-173; Shaw et al., 1983, Gene 23(3):315-330) ou *A. rhizogenes* (Bevan et Chilton, 1982, Annu. Rev. Genet. 16:357-384; Tepfer and Casse-Delbart, 1987, Microbiol. Sci. 4(1), 24-28). De manière préférentielle, la transformation de cellules ou tissus végétaux par *Agrobacterium tumefaciens* est réalisée selon le protocole décrit par Ishida et al. (1996, Nat. Biotechnol. 14(6), 745-750). L'homme du métier fera le choix de la méthode appropriée en fonction de la nature des organismes hôtes à transformer.

La présente invention concerne également un nouvel extrait bactérien comprenant au moins une activité phytase, caractérisé en ce qu'il provient d'une bactérie du genre *Acidocella*. Par extrait bactérien, on entend selon la présente invention une fraction soluble dans l'eau ou dans une solution aqueuse, ladite fraction étant issue du broyage de bactéries et de la séparation par centrifugation des constituants bactériens ainsi libérés en fraction solide et fraction liquide, la fraction liquide comprenant l'ensemble des constituants bactériens solubles. L'activité phytase correspond à la quantité d'acide phytique dégradé et est généralement mesurée par quantification du phosphate inorganique libéré par la réaction enzymatique. En particulier, l'activité phytase est mesurée selon la méthode de Shimizu (1992, Biosci. Biotech, Biochem. 56(8), 1266-1269), en particulier telle que décrite dans l'exemple 2 ci-après. Toutefois, toute méthode permettant de caractériser une activité phytase, soit par la mesure de la diminution de la quantité de substrat, soit par la mesure de l'accumulation des produits issus de la réaction enzymatique, peut être utilisée pour mesurer l'activité phytase de l'extrait bactérien selon l'invention.

L'extrait bactérien selon l'invention est caractérisé en ce qu'il provient d'une bactérie du genre *Acidocella.* Les caractéristiques des bactéries regroupées dans le genre *Acidocella* sont décrites dans Kishimoto et al. (1995, System. Appl. Microbiol. 18, 85-91).

De préférence, l'extrait bactérien selon l'invention exprime une activité phytase dont le pH optimal d'activité est inférieur ou égal à 4. Le pH optimal d'activité correspond au pH auquel l'activité phytase mesurée dans l'extrait bactérien selon l'invention est maximale.

Selon un mode particulier de réalisation de l'invention, l'extrait bactérien comprend une activité phytase possédant les propriétés suivantes:
(a) température optimale = 70°C
(b) pH optimal = 4

Selon un autre mode particulier de réalisation de l'invention, l'extrait bactérien comprend une activité phytase possédant les propriétés suivantes:
(a) température optimale = 60°C
(b) pH optimal = 3,5

Par température optimale et pH optimal, on entend la température et le pH auxquels l'activité phytase mesurée dans l'extrait bactérien selon l'invention est maximale.

Selon un mode particulier de réalisation de l'invention, l'extrait bactérien selon l'invention provient d'une souche bactérienne de l'espèce *Acidocella aminolytica*, en particulier la souche *A. aminolytica* ATCC 51361, ou d'une souche bactérienne de l'espèce *Acidocella facilis,* en particulier la souche *A. facilis* ATCC 35904.

La présente invention concerne également un procédé de préparation dudit extrait bactérien. Ce procédé est **caractérisé en ce qu**'il comprend les étapes de:
(a) mise en culture d'une souche bactérienne du genre *Acidocella*
(b) concentration des bactéries cultivées à l'étape (a)
(c) broyage des bactéries isolées à l'étape (b)
(d) centrifugation du broyat obtenu à l'étape (c)
(e) récupération du surnageant possédant l'activité phytase issu de l'étape (d)

Selon le présent procédé, une souche bactérienne du genre *Acidocella* est mise en culture dans un milieu de culture approprié permettant aux bactéries de se multiplier, et dont le pH est ajusté aux conditions de pH du milieu naturel dans lequel se développe ladite souche bactérienne. De nombreux milieux de culture pour bactéries sont décrits dans la littérature et l'homme du métier saura les sélectionner et en adapter les conditions de pH. A titre d'exemple, un milieu de culture pour bactéries acidophiles, en particulier du genre *Acidocella* est décrit dans Kishimoto et al., 1995, System. Appl. Microbiol. 18, 85-91).

Après mise en culture, les bactéries obtenues sont concentrées. La concentration des bactéries peut s'effectuer par de nombreux moyens, en particulier par centrifugation ou par filtration. Après concentration, les bactéries sont broyées. Le broyage des bactéries consiste à casser les cellules bactériennes. Il peut s'effectuer par différents moyens connus de l'homme du métier. De préférence, le broyage est réalisé par exposition des bactéries à un champ d'ultrasons. Le broyat bactérien est ensuite centrifugé afin de séparer les débris cellulaires non solubles, puis de récupérer le surnageant de centrifugation comprenant les éléments cellulaires solubles, et en particulier, des enzymes exprimant une activité phytase.

Selon un mode particulier de réalisation de l'invention, la souche bactérienne utilisée dans le procédé selon l'invention est une souche bactérienne de l'espèce *Acidocella aminolytica,* en particulier la souche *A. aminolytica* ATCC 51361, ou une souche bactérienne de l'espèce *Acidocella facilis*, en particulier la souche *A. facilis* ATCC 35904.

La présente invention concerne également un procédé de production de la phytase selon l'invention. Selon un mode de réalisation de l'invention, ce procédé est caractérisé en ce qu'il comprend les étapes de:
(a) mise en culture d'une souche bactérienne du genre *Acidocella*
(b) concentration des bactéries cultivées à l'étape (a)
(c) broyage des bactéries isolées à l'étape (b)
(d) centrifugation du broyat obtenu à l'étape (c)
(e) récupération du surnageant possédant l'activité phytase issu de l'étape (d)
(f) purification de la phytase à partir du surnageant récupéré à l'étape (e)

Les étapes (a) à (e) de ce procédé sont communes avec les étapes (a) à (e) du procédé de préparation d'un extrait bactérien décrit ci-dessus. L'étape (f) du présent procédé de production d'une phytase selon l'invention consiste à purifier la phytase à partir du surnageant récupéré à l'étape (e). La purification de la phytase peut se faire par toute technique de séparation des protéines, en particulier les techniques d'électrophorèse et de chromatographie bien connues de l'homme du métier. Afin de parvenir à une phytase purifiée, l'homme du métier saura employer la méthode de mesure de l'activité phytase ci-dessus mentionnée pour identifier la ou les fractions de purification contenant ladite phytase.

De préférence, la souche bactérienne utilisée dans le procédé selon l'invention est une souche bactérienne de l'espèce *Acidocella aminolytica,* en particulier la souche *A. aminolytica* ATCC 51361, ou d'une souche bactérienne de l'espèce *Acidocella facilis,* en particulier la souche *A. facilis* ATCC 35904.

Selon un autre mode de réalisation de l'invention, en particulier lorsque la phytase selon l'invention est sécrétée dans le milieu de culture, ce procédé est caractérisé en ce qu'il comprend les étapes de:
(a) mise en culture d'une souche bactérienne du genre *Acidocella*
(b) récupération du milieu de culture par élimination des bactéries
(c) purification de la phytase à partir du milieu de culture récupéré à l'étape (b)

Selon ce procédé, l'étape de récupération du milieu de culture par élimination des bactéries peut se faire par tout moyen de séparation de fractions solides comprises dans une fraction liquide. En particulier, la filtration et la centrifugation sont des moyens adaptés à la mise en oeuvre de cette étape.

Selon un autre mode de réalisation de l'invention, le procédé de production de la phytase selon l'invention met en oeuvre un organisme hôte transformé selon l'invention, et est caractérisé en ce qu'il comprend les étapes de:
(a) mise en culture d'un organisme hôte transformé selon l'invention
(b) concentration de l'organisme hôte transformé cultivé à l'étape (a)
(c) broyage de l'organisme hôte transformé isolé à l'étape (b)
(d) centrifugation du broyat obtenu à l'étape (c)
(e) récupération du surnageant possédant l'activité phytase issu de l'étape (d)
(f) purification de la phytase à partir du surnageant récupéré à l'étape (e)

L'étape (f) du présent procédé de production d'une phytase selon ce mode de réalisation de l'invention consiste à purifier la phytase à partir du surnageant récupéré à l'étape (e). La purification de la phytase peut se faire par toute technique de séparation des protéines, en particulier les techniques d'électrophorèse et de chromatographie bien connues de l'homme du métier. Afin de parvenir à une phytase purifiée, l'homme du métier saura employer la méthode de mesure de l'activité phytase ci-dessus mentionnée pour identifier la ou les fractions de purification contenant ladite phytase.

De préférence, l'organisme hôte transformé mis en oeuvre dans ce procédé est un microorganisme. En particulier ledit microorganisme peut être un champignon, par exemple des genres *Penicillium, Aspergillus, Chrysosporium,* ou *Trichoderma,* une bactérie, par exemple du genre *Escherichia,* en particulier *E. coli,* du genre *Corynebacterium* ou du genre *Streptomyces,* une levure, en particulier des genres *Saccharomyces, Kluyveromyces*, ou *Pichia,* un baculovirus, ou des cellules végétales.

Selon un autre mode de réalisation de l'invention, en particulier lorsque la phytase selon l'invention est sécrétée dans un milieu de culture, ce procédé est caractérisé en ce qu'il comprend les étapes de:
(a) mise en culture d'un organisme hôte transformé selon l'invention
(b) récupération du milieu de culture par élimination dudit organisme hôte transformé
(c) purification de la phytase à partir du milieu de culture récupéré à l'étape (b)

Selon ce procédé, l'étape de récupération du milieu de culture par élimination de l'organisme hôte transformé peut se faire par tout moyen de séparation de fractions solides comprises dans une fraction liquide. En particulier, la filtration et la centrifugation sont des moyens adaptés à la mise en oeuvre de cette étape.

La présente invention concerne également des compositions enzymatiques comprenant au moins phytase selon l'invention. Selon un mode de réalisation particulier, la composition enzymatique selon l'invention comprend un extrait bactérien selon l'invention.

Par "composition enzymatique", on entend une composition comprenant au moins une phytase selon l'invention, laquelle phytase est associée à des adjuvants divers favorisant sa stabilité et sa conservation. La composition enzymatique selon l'invention peut être sous forme liquide, ladite composition et la phytase qu'elle contient se trouvant alors dans une solution aqueuse, ou sous forme solide, ladite composition et la phytase qu'elle contient se trouvant alors lyophilisée sous forme de poudre.

Selon un mode particulier de réalisation de l'invention, la composition enzymatique comprend, en plus de la phytase selon l'invention, au moins une enzyme additionnelle. Cette enzyme additionnelle peut soit posséder une activité phytase, soit posséder une activité autre que l'activité phytase. Dans le cas où cette enzyme additionnelle possède une activité phytase, ladite activité phytase est de préférence différente et complémentaire de l'activité phytase de la phytase selon l'invention. Dans le cas où cette enzyme additionnelle possède une activité autre que l'activité phytase, elle peut par exemple posséder une activité xylanase, cellulase, β-glucanase, ferulic acide esterase, pullulanase, amidase, phosphatase, ou mannanase.

De manière préférée, lesdites compositions enzymatiques sont destinées à être incorporées dans des aliments pour animaux d'élevage, en particulier des animaux monogastriques, de préférence des Porcs ou des Volailles.

La présente invention concerne également une composition alimentaire comprenant au moins phytase selon l'invention.

Par "composition alimentaire", on entend essentiellement un aliment destiné aux animaux d'élevage, en particulier aux animaux monogastriques, de préférence les Porcs ou les Volailles. Une composition alimentaire selon l'invention est idéalement un aliment destiné aux animaux d'élevage additionné d'une composition enzymatique selon l'invention. La composition alimentaire selon l'invention est donc un aliment pour animaux d'élevage auquel est additionné au moins une composition enzymatique selon l'invention, ledit aliment et ladite composition enzymatique étant mixés de manière à obtenir ladite composition alimentaire.

Selon un mode particulier de réalisation de l'invention, lesdites compositions alimentaires comprennent au moins un organisme hôte transformé selon l'invention. Selon un autre mode particulier de réalisation de l'invention, lesdites compositions alimentaires comprennent au moins une bactérie du genre *Acidocella.* De manière préférentielle, la bactérie du genre *Acidocella* est une bactérie de l'espèce *Acidocella aminolytica,* en particulier la souche *A. aminolytica* ATCC 51361, ou une bactérie de l'espèce *Acidocella facilis,* en particulier la souche *A. facilis* ATCC 35904.

L'invention concerne également des compositions alimentaires comprenant au moins un extrait bactérien ou une phytase selon l'invention.

Avantageusement, les compositions alimentaires selon l'invention sont destinées à être utilisées dans l'alimentation des animaux monogastriques. Selon un mode particulier de réalisation de l'invention, lesdites compositions alimentaires sont destinées à l'alimentation des Porcs. Selon un autre mode particulier de réalisation de l'invention, lesdites compositions alimentaires sont destinées à l'alimentation des Volailles.

La présente invention a également pour objet un procédé de production d'une composition alimentaire telle que décrite ci-dessus.

Selon un mode particulier de réalisation de l'invention, ledit procédé consiste à mettre en culture un organisme hôte selon l'invention ou une bactérie du genre *Acidocella,* à concentrer lesdits organismes hôtes ou lesdites bactéries par tout procédé de concentration connu de l'homme du métier, en particulier la filtration ou la centrifugation, puis à incorporé lesdits organismes hôtes ou lesdites bactéries concentrés dans une composition alimentaire. De manière préférentielle, lorsque le procédé selon l'invention met en oeuvre une bactérie du genre *Acidocella,* celle-ci est une bactérie de l'espèce *Acidocella aminolytica,* en particulier la souche *A. aminolytica* ATCC 51361, ou une bactérie de l'espèce *Acidocella facilis*, en particulier 1a souche *A. facilis* ATCC 35904.

Selon un autre mode particulier de réalisation de l'invention, ledit procédé consiste à isoler un extrait bactérien selon l'invention par le procédé de préparation dudit extrait tel que décrit ci-dessus, puis à incorporer le surnageant produit à l'étape (e) dudit procédé dans une composition alimentaire.

Selon un autre mode particulier de réalisation de l'invention, ledit procédé consiste à produire une phytase selon l'invention par un des procédés de production de ladite phytase tels que décrits ci-dessus, puis à incorporer ladite phytase produite dans une composition alimentaire.

La présente invention concerne également un procédé pour augmenter l'assimilation du phosphate inorganique contenu dans le phytase des aliments à base de végétaux par les animaux monogastriques, **caractérisé en ce que** l'on incorpore une phytase ou une composition enzymatique selon l'invention dans l'alimentation desdits animaux. De manière préférée, ledit procédé est **caractérisé en ce que** l'on alimente lesdits animaux monogastriques avec une composition alimentaire selon l'invention.

L'invention concerne aussi un procédé pour diminuer l'adjonction de phosphore dans l'alimentation des animaux monogastriques, **caractérisé en ce que** l'on alimente lesdits animaux avec une composition alimentaire selon l'invention.

L'invention concerne également un procédé pour diminuer les rejets de phosphore issus de l'alimentation des animaux monogastriques, **caractérisé en ce que** l'on alimente lesdits animaux avec une composition alimentaire selon l'invention.

Les exemples ci-après permettent d'illustrer la présente invention, sans toutefois en limiter la portée.

### Exemple 1: Culture des bactéries du genre Acidocella

Les bactéries du genre *Acidocella* sont des bactéries acidophiles à Gram négatif, qui se développent dans des milieux à pH acide. A titre d'exemple, les bactéries des souches *A. aminolytica* ATCC 51361 et *A. facilis* ATCC 35904 peuvent être employées pour mettre en oeuvre la présente invention. Ces bactéries peuvent être mises en culture selon une méthode similaire. En particulier, elles sont cultivées en conditions aérobies à 30°C et pH 4 dans du milieu composé de peptone et de glucose. Un milieu de culture type est par exemple décrit dans Kishimoto et Tano (1987, J. Gen. Appl. Microbiol. 33, 11-25). Le milieu utilisé pour l'expression de la phytase est exempt de phosphate inorganique, et supplémenté en phytate (0,4%) et en CaC12 (2,2 g/l).

### Exemple 2: Mesure de l'activité phytase

La mesure de l'activité phytase est réalisée en milieu liquide. Elle s'effectue sur des échantillons de culture bactérienne ou sur des extraits bactériens selon l'invention, ou sur des phytases selon l'invention. Elle est basée sur la méthode de Shimizu (1992, Biosci. Biotech. Biochem. 56(8), 1266-1269). Le principe de cette méthode consiste à mesurer la quantité de phosphate inorganique libéré lors de la réaction enzymatique de la phytase avec son substrat (solution de phytate de sodium à 1%). La quantité de phosphate inorganique libéré est mesurée par réaction de celui-ci avec un réactif chromogène (1 volume de sulfate de fer à 10,8% mélangé extemporanément à 4 volumes d'ammonium molybdate 0,012M H₂SO₄). Cette réaction conduit, en milieu fortement acide, à la formation d'un complexe de phosphomolybdate coloré (en présence de Fe²⁺), et la quantité de complexe formé est quantifiée par la mesure au spectrophotomètre de l'absorbance à 700 nm de la solution colorée générée.

Deux réactions distinctes permettent la mesure de l'activité: une première mesure est effectuée au moment de la mise en contact de l'échantillon avec le substrat (temps T0) et une seconde après 60 min d'incubation (temps T60). La variation de l'absorbance entre les temps T0 et T60 (contre les blancs respectifs constitués de tampon à la place de l'échantillon) est proportionnelle à la quantité de phosphate inorganique libéré lors de la réaction enzymatique.

Une gamme étalon de référence est réalisée en mélangeant 250µl de solution de KH₂PO₄ aux concentrations de 0,1mM, 0,5mM, 1mM, 2mM et 4mM dans le tampon désiré à 250µl de TCA et en révélant par 450µl de réactif chromogène. Une unité enzymatique est définie comme la quantité d'enzyme libérant une micromole de phosphate inorganique par minute (à une température et un pH donné).

### Exemple 3: Isolement d'un extrait bactérien contenant une activité phytase

Après culture, les bactéries du genre *Acidocella* sont concentrées par centrifugation pendant 15 min à 5000g et 4°C. Le surnageant est éliminé, et les bactéries ainsi concentrées sont ensuite remises en suspension dans un tampon formate (100 mM)-CaCl2 (1 mM) à pH 3,5 de manière a les concentrer environ 100 fois par rapport à leur concentration initiale dans le milieu de culture. Les bactéries concentrées sont ensuite broyées à l'aide d'un appareil "Cell disrupter" (Cell-d) utilisé à raison de deux passages dans un flux d'une pression de 2,5 kbar. Les broyats bactériens sont ensuite centrifugés pendant 15 min à 5000g et 4°C et le surnageant, constituant l'extrait bactérien, est récupéré et son activité phytase mesurée.

### Exemple 4: Caractéristiques des phytases isolées selon l'invention

### 4.1. Activité phytase en fonction de la température

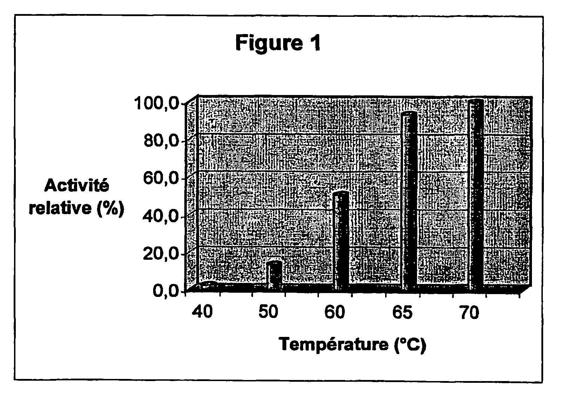

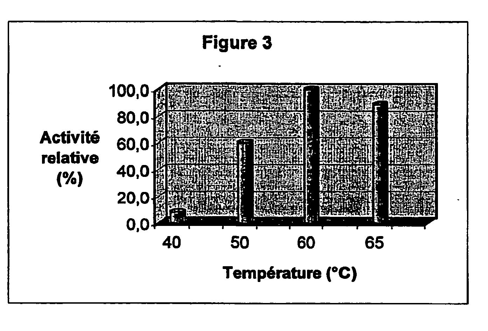

L'activité des phytases selon l'invention a été déterminée à différentes températures. Les résultats de ces mesures sont présentés pour les bactéries *Acidocella aminolytica* ATCC 51361 (Figure 1), et *Acidocella facilis* ATCC 35904 (Figure 3). Les températures optimales d'activité sont de 70°C et 60°C respectivement pour les bactéries *Acidocella aminolytica* ATCC 51361, *Acidocella facilis* ATCC 35904.

### 4.2. Activité phytase en fonction du pH

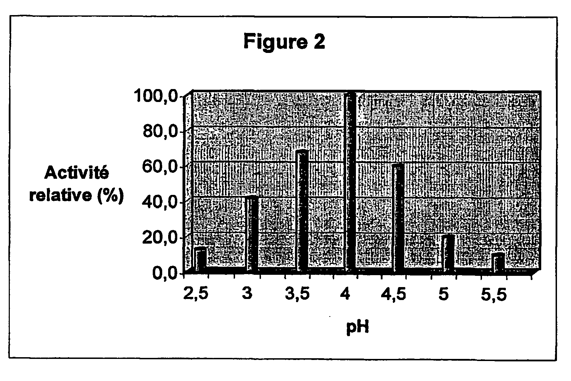

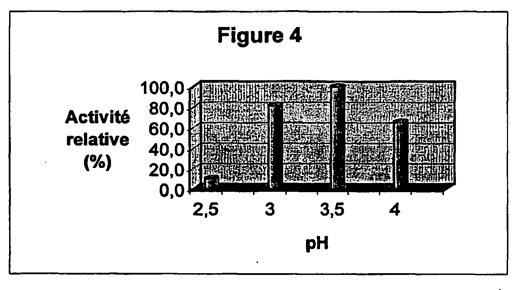

L'activité des phytases selon l'invention a été déterminée à différents pH. Les résultats de ces mesures sont présentés pour les bactéries *Acidocella aminolytica* ATCC 51361 (Figure 2), et *Acidocella facilis* ATCC 35904 (Figure 4). Les pH optimum d'activité sont de 4 et 3,5 respectivement pour les bactéries *Acidocella aminolytica* ATCC 51361, *Acidocella facilis* ATCC 35904.

### Exemple 5: Purification d'une phytase selon l'invention

### 5.1. Préparation d'un extrait brut

Les cellules d'une souche bactérienne du genre *Acidocella* sont centrifugées puis lavées 3 fois en tampon MES pH 6,5 (25 mM) contenant 1 mM CaCl₂ afin d'éliminer le phytate du milieu et le phosphate inorganique (accumulé lors de la croissance). La suspension cellulaire ainsi obtenue est ensuite cassée au "Cell disrupter" (1,5 kbar), puis le pH est abaissé à 5,5 (par ajout d'acide chlorhydrique à 3,7%).

L'extrait ainsi obtenu est centrifugé à 20 000 g pendant 1 heure. Le surnageant est ensuite filtré sur une membrane de porosité 0,45 µm.

### 5.2. Chromatographie d'échange d'anions

Le surnageant est passé sur une colonne de type POROS 20 HQ (4,6/ 100 mm : 1,7 ml de gel). L'élution est réalisée avec un tampon MES pH 5,5 (25 mM) 1 mM CaCl₂, 1M NaCl. L'activité phytase (50% de l'activité injectée) est trouvée dans la fraction du volume mort.

### 5.3. Chromatographie d'interaction hydrophobe

La fraction récupérée en fin de chromatographie d'échange d'anion est amenée à 1,5 M (NH₄)₂SO₄. Cette solution est alors déposée sur une colonne de type POROS 20 HP2 (HQ 4,6/100 mm : 1,7 ml de gel) préalablement équilibrée avec un tampon MES pH 5,5 (25 mM), 1 mM CaCl2, 1,5 M (NH₄)₂SO₄. Les protéines sont ensuite éluées par un gradient de 1,5 à 0 M (NH₄)₂SO₄ en 20 volumes de colonne.

L'activité phytase est éluée à 0,95M (NH₄)₂SO₄ dans 3 fractions de 2 ml. Ces fractions sont réunies puis dialysées contre 5 litres de tampon MES pH 5,5 (20mM), 1 mM CaCl₂ à 4°C durant une nuit.

### 5.4. Chromatographie échangeuse de cations

La solution dialysée est injectée sur une colonne de type POROS 20 HS (4,6/ 100 mm : 1,7 ml de gel) préalablement équilibrée avec un tampon MES pH 5,5 (20mM), 1 mM CaCl₂. Les protéines sont éluées par un gradient NaCl de 0 à 1 M en 20 volumes de colonnes.

La protéine à activité phytase est éluée à une concentration en NaCl de 0,35 M.

### 5.5. Filtration sur gel

Les fractions obtenues lors de l'étape précédente ont été réunies, concentrées et déposées sur une colonne SUPEROSE 12 HR 10/ 30 (24 ml, Pharmacia®) préalablement équilibrée avec du tampon MES pH 5,5 (25mM), 1 mM CaCl₂, 0,1 M NaCl. Les protéines ont été éluées avec 24 ml du tampon à un débit de 0,5 ml/ min. Des fractions de 0,5 ml ont été récoltées. L'activité phytase est détectée dans trois fractions entre 14 à 15 ml de tampon d'élution (Tableau 1).

**Tableau 1: Activité dans les fractions d'intérêt après l'étape de filtration sur gel**

| Fractions | Fluorescence Test "MUFp" (RFU/min) |
|---|---|
| 1 | 0 |
| 2 | 2240 |
| 3 | 2690 |
| 4 | 1134 |
| 5 | 0 |

Les trois fractions qui présentent une activité phytase ont été concentrées et déposées sur un gel SDS-PAGE de 14,4%. Ce gel révèle une bande principale d'environ 63 kDa.

### Exemple 6: Microséquençage de peptides internes

La bande d'intérêt (à environ 63 kDa) a été découpée directement sur le gel de polyacrylamide. Le colorant contenu dans le gel est en partie retiré par 2 lavages dans 50% acétonitrile/50 mM Tris-HCl pH 8,6. La protéine, en gel de polyacrylamide, est digérée dans 400 µl de tampon Tris-HCl 50 mM pH 8,6 / 0,03% de SDS à 35°C pendant 18 heures en présence de 0,4 µg d'endolysine-C (Sequencing grade de Roche).

Les peptides obtenus sont séparés par HPLC sur une colonne en ligne de DEAE-C18 de 2,1 mm de diamètre. Le gradient de séparation est un gradient de 2 à 45% d'acétonitrile/TFA 0,1%.

Quatre peptides ont été purifiés, puis séquencés par la méthode d'Edman à l'aide d'un séquenceur Applied Biosystems 473A.

**Tableau 2: Séquences des peptides internes obtenus**

| Peptides | Séquence |
|---|---|
| Peptide 1 | KVINFLMRQPDWK |
| Peptide 2 | KNTAIIITYDDSDGGY |
| Peptide 3 | KHLVIIYGENVSFDHY |
| Peptide 4 | KMEGQNIGDLLNAK |

### Exemple 7: Clonage du gène codant la phytase

### 7.1. Définition d'une sonde

Sur la base des séquences des peptides internes, plusieurs oligonucléotides dégénérés ont été synthétisés afin d'amplifier par PCR un fragment du gène d'intérêt.
Oligo10 : ATDATDATNGCNGTRTTYTT
Oligo12 : TCRTCRTASGTGATGATGATSGCSGTRTTYTT
Oligo22 : AARATGGARGGNCARAAYATHGG
Oligo23 : ATGGAAGGCCAGAAYATCGGCGA
Oligo25 : ATCGGCGAYCTBCTBAAYGCCAA

Des combinaisons des amorces Oligo10 et Oligo12 avec les trois amorces Oligo22, 23, et 25 ont été testées, et le couple d'amorces Oligo12 et Oligo22 a permis d'isoler le fragment de 500 bp à partir de l'ADN génomique d'une souche du genre *Acidocella*. Après purification, ce fragment d'ADN a été séquencé.

L'analyse de cette séquence a permis de mettre en évidence dans ce fragment une séquence nucléique codant pour le peptide 1 suggérant que le produit PCR séquencé correspond effectivement à la protéine d'intérêt qui a fait l'objet du microséquençage.

Sur la base de la séquence obtenue à partir du produit PCR d'environ 500 pb, de nouvelles amorces Oligo29 et Oligo30 ont été définies afin de disposer d'un jeu d'amorces spécifiques non dégénérées du gène d'intérêt.
Oligo29 : TTCATGGGTGGCTTCGATC
Oligo30 : GCTGCCGCATCAGGAAGTTG

### 7.2. Southern blot

Le produit PCR d'environ 500 bp obtenu par amplification avec les amorces Oligo12 et Oligo22 a été utilisé comme sonde dans des expériences de Southern Blot.

Afin de marquer la sonde, 10 ng de produit PCR purifié ont été marqués par "random priming" à l'aide du kit Labelling Q-BIOgene.

10 µg d'ADN génomique ont été digérés respectivement par *Eco* RI, *Sac* II ou *Bam* HI puis déposés sur un gel agarose 1%. Après migration, l'ADN a été transféré par capillarité sur une membrane de nylon (BIODYNE® Plus membrane, Pall Gelman).

Une préhybridation est effectuée pendant 4h à 60°C avec un tampon 5x SSC, 5x Dehardt, 1% SDS,100 µg/ml ADN dénaturé de poisson.

L'hybridation est ensuite réalisée pendant 16h à 60°C avec un tampon contenant 10 ml de tampon de préhybridation et 10 ng de la sonde à 4,08 10⁸ cpm/µg (comptage au compteur à scintillations à sec, c'est-à-dire en absence de scintillant).

Plusieurs phases de lavage ont ensuite suivi:
2 lavages : 2x SCC + 0.1% SDS pendant 3 min à 20°C
2 lavages : 0,5x SCC + 0.1% SDS pendant 3 min à 20°C
2 lavages : 0,4x SCC + 0.1% SDS pendant 15 min à 50°C

Le gel a été révélé sur film après une exposition de 24h à -80°C.

Ces expériences ont permis de montrer que *Sac* II coupe dans la portion du gène utilisée comme sonde, mais qu'il n'y a pas de site *Eco* RI ni *Bam* HI dans cette séquence. Les digestions *Eco* RI et *Bam* HI libèrent respectivement un fragment d'environ 4 kb et un fragment de taille supérieur à 9 kb sur lesquels s'hybride la séquence cible.

Sachant, sur la base d'études biochimiques, que la protéine d'intérêt doit être codée par un gène d'environ 1,6 kb, une banque d'ADN génomique par digestion totale *Eco* RI a été construite.

### 7.3. Construction d'une banque génomique

Environ 30 µg d'ADN génomique d'une souche du genre *Acidocella* ont été digérés par *Eco* RI. Après migration sur gel d'agarose 0,7%, les fragments dont la taille est comprise entre 3 et 4 kb ont été purifiés à l'aide du kit QIAEX II gel extraction kit (Qiagen) et dessalés (microbio spin 6, Biorad). Les fragments dont la taille est comprise entre 2,5 et 3 kb ainsi que ceux dont la taille est comprise 4 et 5 kb ont également été purifiés.

Les fragments purifiés ont été insérés dans un vecteur pBlueScript KS (II+) à l'aide de la T4 DNA ligase (QBIOgene). Les produits de la ligation réalisée avec les fragments dont la taille est comprise entre 3 et 4 kb ont été utilisés pour transformer la souche MC 1061 d'*E*. *coli* par électroporation. Après transformation, les cellules sont reprises en SOC (2% tryptone, 0,5% d'extrait de levure, 0,05% NaCl, 2,5 mM KCl, 10 mM MgCl₂, 20 mM glucose) et placées 1 heure à 37°C avant d'être étalées sur un milieu LB supplémenté en ampicilline (100 µg/ml). Les boites de Pétri sont alors incubées une nuit à 37°C.

### 7.4. Criblage de la banque génomique

383 colonies distinctes ont alors été criblées par PCR en utilisant le jeu d'amorces Oligo29 et Oligo30.

Parmi ces clones, 4 ont permis l'obtention d'un produit PCR d'environ 400 pb. Deux des clones permettant l'amplification d'un fragment de taille attendue ont été utilisés pour réaliser une mini-préparation de plasmide (à l'aide du kit Qiagen).

### 7.5. Séquençage des inserts génomiques

Les deux plasmides identifiés ont été digérés par *Eco* RI et *Eco* RV. Le séquençage de leurs inserts a montré que ces deux plasmides portaient le même insert. L'analyse des séquences a permis la mise en évidence d'une phase ouverte de lecture de 1665 pb correspondant à une protéine de 554 acides aminés. En outre, les séquences codant les 4 peptides internes séquencés à l'exemple 6 ont été retrouvés dans cette phase de lecture.

### Exemple 8: Sous-clonage du gène identifié

La séquence contenant la phase ouverte de lecture (dénommée ORF281) identifiée dans l'insert génomique cloné à l'exemple 7 a été amplifiée par PCR avec les oligonucléotides 5'ORF281 et 3'ORF281.
5'ORF281 5' TAA CGA TAA CAT ATG AAT ATC CGC CGG GCT CT 3'
3'ORF281 5' ATA TCT GAT AAG CTT TTA TTC GGC GTG CGC GGC 3'

Le produit PCR a été purifié et digéré par les enzymes de restriction *Nde* I et *Hind* III puis sous-cloné dans le vecteur pETCm digéré par les même enzymes. Le vecteur obtenu est appelé pETCm281. L'insert ORF281 a été séquencé afin de vérifier que la séquence clonée est identique à celle déterminée à partir des inserts génomiques préalablement clonés.

Le vecteur pETCm comporte un gène de résistance au chloramphénicol. Son utilisation, et donc celle de la résistance au chloramphénicol comme marqueur de sélection, permet l'expression *in vivo* dans une souche de *E. coli* résistante à 1a kanamycine : la souche BL21 *appA* (phytase -).

### Exemple 9: Expression du gène sous-cloné dans E. coli

### 9.1. Préparation des souches E. coli recombinantes

Des essais d'expression *in vivo* ont été faits dans la souche *E. coli* BL21 *appA*. Cette souche n'exprime plus de phytase endogène du fait d'une insertion d'un gène de résistance à la kanamycine dans le gène *appA* codant pour la phytase de *E. coli.* Un vecteur témoin a également été préparé avec le gène TEM codant la β-lactamase de *E*. *coli.* Le gène TEM a aussi été cloné dans le vecteur pETCm, et le vecteur obtenu a été nommé pETCmTEM.

La souche de *E. coli* BL21 *appA* a été transformée par les vecteurs pETCmTEM ou pETCm281. Des colonies isolées ont été sélectionnées sur milieu LB additionné de 60 mg/L de kanamycine et de 20 mg/L de chloramphénicol. Une colonie de chaque type a été mise en culture dans du milieu LB additionné de kanamycine et de chloramphénicol aux même concentrations. Ces cultures ont été utilisées pour préparer des stocks glycérolés (un volume de cellules pour un volume de glycérol 40%) à partir desquels sont ensemencées les cultures destinées à faire les tests d'expression. Ces stocks glycérolés sont conservés à-80°C.

### 9.2. Tests d'expression

La souche *E*. *coli* BL21 *appA* pETCm281 a été cultivée en milieu M98 (milieu Terrific Broth modifié (avec Glucose) contenant, pour 1 litre, 12g de Bacto Tryptone, 24g d'extrait de levure, 9,4g de K₂HPO₄, et 2,2g de KH₂PO₄, pH 7.2, autoclavé, auxquels 20 mL de glucose (50%) par litre de milieu sont ajouté avant utilisation) en parallèle avec la souche *E. coli* BL21 *appA* pETCmTEM. L'expression est induite par l'ajout de 0,25 mM d'IPTG, et réalisée à deux températures : 30 et 37°C.

Après 3 heures d'induction, les cultures sont centrifugées. L'activité phytase est dosée sur chaque culot resuspendu dans du tampon formate pour atteindre une concentration finale de 5 mg/mL de cellules sèches. Aucune activité a été trouvée pour la souche contenant pETCm281 ainsi que la souche contenant pETCmTEM quelles que soient les conditions de culture.

Des aliquotes des culots des cultures induites et non induites contenant pETCm281 ont été alors traités aux ultrasons puis centrifugés. Le surnageant représente la fraction des protéines solubles et le culot les protéines insolubles. Ces deux fractions ont été déposées sur gel dénaturant SDS-PAGE.

Il apparaît clairement une bande protéique de PM 63 kDa dans la fraction insoluble de la souche *E. coli* BL21 *appA* pETCm281 induite à l'IPTG. Aucune bande correspondante n'est trouvée dans le témoin négatif non induit. L'ORF281 est donc bien exprimée chez *E. coli.* Toutefois, la protéine correspondante est insoluble et vraisemblablement inactive sous cette forme.

### 9.3 Solubilisation et renaturation des corps d'inclusion

Une série d'étapes visant à solubiliser puis de renaturer la protéine codée par l'ORF281 a été réalisée afin de déterminer si cette protéine possède ou non une activité phytase.

La souche *E*. *coli* BL21 *appA* pETCm281 est cultivée en milieu M98 puis induite avec 0,25 mM d'IPTG lorsque la DO à 600 nm atteint 0,4. Après 3 heures d'induction, la culture est centrifugée (DO finale autour de 1). Le culot est repris dans du tampon Tris-HCl 20 mM , pH 8,0 à raison de 4 mL pour 100 mL de culture. Les cellules sont traitées aux ultrasons à 4°C : 4 cycles de 10 secondes à une puissance de 500 Watt. La DO₆₀₀ passe de 14 à 3. La suspension est ensuite centrifugée 10 minutes à 16000g, puis le surnageant est éliminé.

Le culot est resuspendu dans 5 ml pour 100 ml de culture de tampon de solubilisation : Tris-HCl 20 mM pH 8, Urée 8M, NaCl 0,5 M, 2-mercaptoéthanol 1 mM. La suspension est traitée aux ultrasons à raison de 4 cycles de 10 secondes à une puissance de 500 Watt, puis agitée 1 heure à température ambiante. 30 ml de cette suspension sont ensuite dialysés à 4°C durant 16 heures contre 3 litres de tampon de renaturation. MES 25 mM pH 6,5, 0,5 M NaCl, 1 mM Mercaptoéthanol (Cutt-off 10 kDa). Une seconde dialyse est réalisée contre 3 litres de tampon de renaturation MES 25 mM pH 5,5 , 0,5 M NaCl, 1 mM Mercaptoéthanol (Cutt-off 10 kDa) durant 5 heures. La suspension est ensuite centrifugée 10 minutes à 16000 g puis conservée à 4°C avant dosage enzymatique.

Le même protocole de culture, de solubilisation et de renaturation, a été appliqué à la souche *E. coli* BL21 *appA* pETCmTEM.

### 9.4. Activité des corps d'inclusion

Les suspensions des protéines renaturées produites par les souches *E*. *coli* BL21 *appA* pETCm281 et pETCmTEM ont été concentrées 10 fois sur Nanosep™ 10K Omega (Pall). L'activité phytase est mesurée sur ces solutions concentrées suivant un protocole standard (à pH 3,5). Ce test a été réalisé en double.

| | Activité mU/ml |
|---|---|
| E. coli BL21 appA pET-TEM. Test 1 | 0 |
| E coli BL21 appA pET-TEM. Test 2 | 0 |
| E. coli BL21 appA pET281. Test 1 | 100 |
| E. coli BL21 appA pET281. Test 2 | 100 |

Seule une activité phytase est détectée dans la souche *E*. *coli* BL21 *appA* pETCm281. Cette activité est confirmée par un dosage pMUF. En utilisant cette méthode fluorométrique, aucune activité n'est détectée dans la souche *E*. *coli* BL21 appA pETCmTEM, bien que ce dosage soit cinq fois plus sensible que la méthode colorimétrique généralement utilisée.

### 9.5. Purification des corps d'inclusion

La fraction resolubilisée des corps d'inclusion de la souche *E. coli* BL21 *appA* pETCm281 a été déposée sur une colonne POROS 20 HS (4,6/ 100 mm : 1,7 ml de gel) préalablement équilibrée avec un tampon 20mM MES pH 5,5, 1 mM CaCl₂. Les protéines sont éluées par un gradient NaCl de 0 à 1 M en 20 volumes de colonnes.

L'activité phytase est éluée à une concentration en NaCl de 0,35 M. Ce résultat est en tout point identique à celui obtenu à l'exemple 5.4. avec la phytase native.

En parallèle, un extrait total (protéines solubles et insolubles) de *E. coli* BL21 *appA* pETCmTEM a été déposé sur la même colonne et les protéines ont été éluées dans les mêmes conditions. Aucune activité phytase n'a pu être détectée dans les fractions éluées entre 0,15 et 0,45 M NaCl.

### 9.6. Gel SDS-PAGE

La fraction présentant une activité phytase, issue de la colonne échangeuse de cations, a été déposée sur un gel SDS-PAGE (10% acrylamide) après concentration d'un facteur 10 sur Nanosep™. Sur le même gel, un lysat de cellules (induites à l'IPTG) de *E*. *coli* BL21 *appA* pETCm281 et *E*. *coli* BL21 *appA* pETCmTEM ont également été déposés.

Il apparaît encore clairement que seul le lysat de *E. coli* BL21 *appA* pETCm281 contient une bande de PM 63 kDa. Dans la fraction purifiée sur colonne et présentant une activité phytase, une bande de PM 61-63 kDa est présente et majoritaire.

Ces résultats montrent donc, après sous-clonage dans *E. coli* BL21 *appA*, que l'ORF281 code bien pour une phytase.

### Exemple 10: Caractéristiques de la phytase recombinante purifiée

### 10.1. Activité phytase en fonction de la température

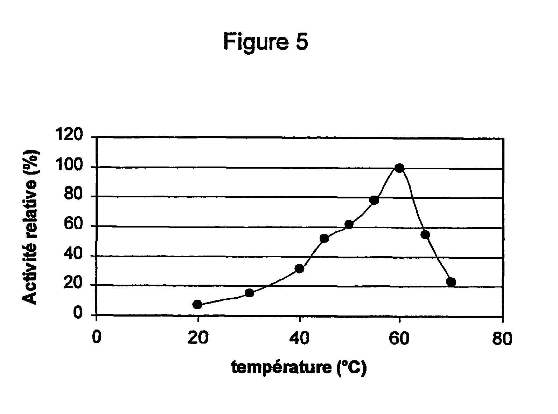

L'activité de la phytase contenue dans la fraction purifiée à l'exemple 5.5 et codée par l'ORF281 a été déterminée à différentes températures. Les résultats de ces mesures sont présentés dans la figure 5. La température optimale d'activité mesurée est de 60°C. Le dosage a été réalisé à pH 3.

### 4.2. Activité phytase en fonction du pH

L'activité de la phytase contenue dans la fraction purifiée à l'exemple 5.5 et codée par l'ORF281 a également été déterminée à différents pH. Les résultats de ces mesures sont présentés dans la figure 5. Le pH optimum d'activité est de 3,5. Le dosage a été réalisé à 60°C, avec la succession de tampons suivants: Glycine-HCl (pour les pH de 2 à 2,5), Formate (pH de 2,5 à 4), Acétate (pH de 4 à 5), et MES (pH de 5 à 6).

### Exemple 11: Recherche de séquences homologues

La séquence contenant la phase ouverte de lecture identifiée dans le fragment génomique cloné à l'exemple 7 a été utilisée comme sonde pour rechercher des séquences homologues parmi les microorganismes des genres *Acidocella* et *Acidiphilium*.

Les souches testées appartiennent aux espèces *Acidocella aminolytica*, *Acidocella facilis, Acidiphilium angustrum*, et *Acidiphilium multivorum*.

10 ng de produit PCR purifié ont été marqués par "random priming" à l'aide du kit Labelling Q-BIOgene. 10 µg d'ADN génomique des différentes souches à tester ont été digérés respectivement par *Eco* RI *ou Eco* RV puis déposés sur deux gels agarose 1%. Après migration, l'ADN a été transféré par capillarité sur deux membranes de nylon (BIODYNE® Plus membrane, Pall Gelman).

Une préhybridation a été menée pendant 4 h à 60°C avec un tampon 5x SSC, 5x Dehardt, 1% SDS, 100 µg/ml ADN dénaturé de poisson.

L'hybridation est ensuite réalisée pendant 16h à 60°C avec un tampon contenant 10 ml de tampon de préhybridation et 10 ng de la sonde à respectivement 7.10⁸ et 9.10⁸ cpm/µg pour la membrane 1 (EcoRI) et la membrane 2 (EcoRV) (comptage au compteur à scintillations à sec, c'est-à-dire en absence de scintillant).

Plusieurs phases de lavage ont ensuite suivi:
2 lavages : 2x SCC + 0.1% SDS pendant 3 min à 20°C
2 lavages : 0,5x SCC + 0.1% SDS pendant 3 min à 20°C
2 lavages : 0,4x SCC + 0.1% SDS pendant 15 min à 50°C

Les membranes ont été révélées sur film après une exposition de 65h à -80°C.

Aucune différence significative n'a été observée entre les deux autoradiographies.

Les résultats montrent la présence, chez toutes les souches du genre *Acidocella* testées, d'une hybridation sélective de la sonde contenant la phase ouverte de lecture identifiée à l'exemple 7. Ces résultats suggèrent qu'il existe une homologie de séquence significative entre des gènes des bactéries du genre *Acidocella* et la phase ouverte de lecture identifiée codant pour une phytase. En outre, ils suggèrent fortement que ces gènes codent également pour des phytases.

A l'inverse, aucune hybridation n'a été obtenue avec la sonde au sein des microorganismes du genre *Acidiphilium.* Cette observation est en accord avec l'absence de détection d'une activité phytase par des méthodes microbiologiques chez les microorganismes de ce genre.

## Revendications

1. Polynucléotide isolé codant pour une phytase, **caractérisé en ce qu'**il est sélectionné dans le groupe consistant en :
(a) un polynucléotide isolé codant pour la phytase décrite par l'identificateur de séquence SEQ ID NO : 2
(b) un polynucléotide isolé s'hybridant au polynucléotide selon (a) dans des conditions stringentes
(c) un polynucléotide isolé présentant au moins 70% d'identité avec le polynucléotide selon (a)
ladite phytase ayant un pH optimal d'activité inférieur ou égal à 4.

2. Polynucléotide selon la revendication 1, **caractérisé en ce qu'**il est représenté par l'identificateur de séquence SEQ ID NO : 1.

3. Polynucléotide selon l'une des revendications 1 ou 2, **caractérisé en ce qu'**il provient d'une bactérie du genre *Acidocella*

4. Polynucléotide isolé comprenant un polynucléotide selon l'une des revendications 1 à 3.

5. Phytase, **caractérisée en ce qu'**elle est codée par un polynucléotide selon l'une des revendications 1 à 4, ladite phytase ayant un pH optimal d'activité inférieur ou égal à 4.

6. Phytase selon la revendication 5, **caractérisée en ce qu'**elle est sélectionnée parmi le groupe consistant en :
(a) la phytase représentée par l'identificateur de séquence SEQ ID NO : 2
(b) une phytase présentant au moins 70% d'identité avec la phytase selon (a)
(c) un fragment biologiquement actif d'une phytase selon (a) ou (b)

7. Phytase selon l'une des revendications 5 ou 6, **caractérisée en ce qu'**elle provient d'une bactérie du genre *Acidocella*

8. Gène chimère comprenant au moins, liés entre eux de manière opérationnelle :
(a) un promoteur fonctionnel dans un organisme hôte
(b) un polynucléotide selon l'une des revendications 1 à 4
(c) un élément terminateur fonctionnel dans un organisme hôte.

9. Gène chimère selon la revendication 8, **caractérisé en ce qu'**il comprend, en plus, un peptide signal ou un peptide de transit fonctionnel dans ledit organisme hôte.

10. Vecteur d'expression ou de transformation contenant un gène chimère selon l'une des revendications 8 ou 9.

11. Vecteur selon la revendication 10, **caractérisé en ce qu'**il est un plasmide, un phage ou un virus.

12. Organisme hôte transformé contenant un vecteur selon la revendication 10.

13. Organisme hôte selon la revendication 12, **caractérisé en ce qu'**il s'agit d'un microorganisme.

14. Organisme hôte selon la revendication 13, **caractérisé en ce que** le microorganisme est sélectionné parmi les bactéries, les champignons, les levures, ou les virus

15. Organisme hôte selon la revendication 14, **caractérisé en ce que** le microorganisme est une bactérie sélectionnée parmi les genres *Corynebacterium, Streptomyces,* et *Escherichia*, en particulier *E*. *coli*.

16. Organisme hôte selon la revendication 14, **caractérisé en ce que** le microorganisme est un champignon sélectionné parmi les genres *Penicillium*, *Aspergilles*, *Chrysosporium*, et *Trichoderma*

17. Organisme hôte selon la revendication 14, **caractérisé en ce que** le microorganisme est une levure sélectionnée parmi les genres *Saccharomyces, Kluyveromyces*, et *Pichia*

18. Organisme hôte selon la revendication 12, **caractérisé en ce qu'**il s'agit d'une cellule végétale, d'une plante, ou une partie de plante

19. Extrait bactérien comprenant au moins une activité phytase, **caractérisé en ce qu'**il provient d'une bactérie du genre *Acidocella* et dont le pH optimal d'activité de ladite activité phytase est inférieur ou égal à 4

20. Extrait bactérien selon la revendication 19, **caractérisé en ce que** l'activité phytase possède les propriétés suivantes :
(a) température optimale = 70°C
(b) pH optimal = 4

21. Extrait bactérien selon la revendication 19, **caractérisé en ce que** l'activité phytase possède les propriétés suivantes :
(a) température optimale = 60°C
(b) pH optimal= 3, 5

22. Procédé de préparation d'un extrait bactérien selon l'une des revendications 19 à 21, **caractérisé en ce qu'**il comprend les étapes de :
(a) mise en culture d'une souche bactérienne du genre *Acidocella*
(b) concentration des bactéries cultivées à l'étape (a)
(c) broyage des bactéries isolées à l'étape (b)
(d) centrifugation du broyat obtenu à l'étape (c)
(e) récupération du surnageant possédant l'activité phytase issu de l'étape (d)

23. Procédé de production d'une phytase selon l'une des revendications 5 à 7, **caractérisé en ce qu'**il comprend les étapes de :
(a) mise en culture d'une souche bactérienne du genre *Acidocella*
(b) concentration des bactéries cultivées à l'étape (a)
(c) broyage des bactéries isolées à l'étape (b)
(d) centrifugation du broyat obtenu à l'étape (c)
(e) récupération du surnageant possédant l'activité phytase issu de l'étape (d)
(f) purification de la phytase à partir du surnageant récupéré à l'étape (e)

24. Procédé de production d'une phytase selon l'une des revendications 5 à 7, **caractérisé en ce qu'**il comprend les étapes de :
(a) mise en culture d'une souche bactérienne du genre *Acidocella*
(b) récupération du milieu de culture par élimination des bactéries
(c) purification de la phytase à partir du milieu de culture récupéré à l'étape (b)

25. Procédé de production d'une phytase selon l'une des revendications 5 à 7, **caractérisé en ce qu'**il comprend les étapes de :
(a) mise en culture d'un organisme hôte transformé selon l'une des revendications 12 à 18
(b) concentration de l'organisme hôte transformé cultivé à l'étape (a)
(c) broyage de l'organisme hôte transformé isolé à l'étape (b)
(d) centrifugation du broyat obtenu à l'étape (c) (e) récupération du surnageant possédant l'activité phytase issu de l'étape (d) (f) purification de la phytase à partir du surnageant récupéré à l'étape (e)

26. Procédé de production d'une phytase selon l'une des revendications 5 à 7, **caractérisé en ce qu'**il comprend les étapes de :
(a) mise en culture d'un organisme hôte transformé selon l'une des revendications 12 à 18.
(b) récupération du milieu de culture par élimination dudit organisme hôte transformé
(c) purification de la phytase à partir du milieu de culture récupéré à l'étape (b)

27. Composition enzymatique, **caractérisée en ce qu'**elle comprend au moins un extrait bactérien selon l'une des revendications 19 à 21.

28. Composition enzymatique, **caractérisée en ce qu'**elle comprend au moins une phytase selon l'une des revendications 5 à 7.

29. Composition alimentaire, **caractérisée en ce qu'**elle comprend au moins un organisme hôte transformé selon l'une des revendications 12 à 18.

30. Composition alimentaire, **caractérisée en ce qu'**elle comprend au moins une bactérie du genre *Acidocella* exprimant une phytase selon l'une des revendications 5 à 7.

31. Composition alimentaire selon la revendication 30, **caractérisée en ce que** la bactérie est sélectionnée parmi les espèces *Acidocella aminolytica* et *Acidocella facilis*.

32. Composition alimentaire, **caractérisée en ce qu'**elle comprend au moins un extrait bactérien selon l'une des revendications 19 à 21.

33. Composition alimentaire, **caractérisée en ce qu'**elle comprend au moins une phytase selon l'une des revendications 5 à 7.

34. Utilisation d'une composition alimentaire selon l'une des revendications 29 à 33 pour l'alimentation des animaux monogastriques.

35. Utilisation d'une composition alimentaire selon la revendication 34, **caractérisée en ce qu'**elle est destinée à l'alimentation des porcs.

36. Utilisation d'une composition alimentaire selon la revendication 34, **caractérisée en ce qu'**elle est destinée à l'alimentation des volailles.

37. Procédé de production d'une composition alimentaire selon la revendication 33, **caractérisé en ce qu'**il comprend les étapes de :
(a) mise en culture d'un organisme hôte selon l'une des revendications 12 à 18
(b) concentration de l'organisme hôte cultivé à l'étape (a)
(c) incorporation de l'organisme hôte isolé à l'étape (b) dans ladite composition alimentaire.

38. Procédé de production d'une composition alimentaire selon l'une des revendications 30 ou 31, **caractérisé en ce qu'**il comprend les étapes de :
(a) mise en culture d'une souche bactérienne du genre *Acidocella*
(b) concentration des bactéries cultivées à l'étape (a)
(c) incorporation des bactéries isolées à l'étape (b) dans ladite composition alimentaire

39. Procédé de production d'une composition alimentaire selon la revendication 32, **caractérisé en ce qu'**il comprend les étapes de :
(a) mise en culture d'une souche bactérienne du genre *Acidocella*
(b) concentration des bactéries cultivées à l'étape (a)
(c) broyage des bactéries isolées à l'étape (b)
(d) centrifugation du broyat obtenu à l'étape (c)
(e) récupération du surnageant possédant l'activité phytase issu de l'étape (d)
(f) incorporation du surnageant récupérée à l'étape (e) dans ladite composition alimentaire.

40. Procédé de production d'une composition alimentaire selon la revendication 33, **caractérisé en ce qu'**il comprend les étapes de :
(a) mise en culture d'une souche bactérienne du genre *Acidocella*
(b) concentration des bactéries cultivées à l'étape (a)
(c) broyage des bactéries isolées à l'étape (b)
(d) centrifugation du broyat obtenu à l'étape (c)
(e) récupération du surnageant possédant l'activité phytase issu de l'étape (d)
(f) purification de la phytase à partir du surnageant récupéré à l'étape (e)
(g) incorporation de la phytase purifiée à l'étape (f) dans ladite composition alimentaire

41. Procédé de production d'une composition alimentaire selon la revendication 33, **caractérisé en ce qu'**il comprend les étapes de :
(a) mise en culture d'une souche bactérienne du genre *Acidocella*
(b) récupération du milieu de culture par élimination des bactéries
(c) purification de la phytase à partir du milieu de culture récupéré à l'étape (b)
(d) incorporation de la phytase purifiée à l'étape (c) dans ladite composition alimentaire

42. Procédé de production d'une composition alimentaire selon la revendication 33, **caractérisé en ce qu'**il comprend les étapes de :
(a) mise en culture d'un organisme hôte transformé selon l'une des revendications 12 à 18
(b) concentration de l'organisme hôte transformé cultivé à l'étape (a)
(c) broyage de l'organisme hôte transformé isolé à l'étape (b)
(d) centrifugation du broyat obtenu à l'étape (c)
(e) récupération du surnageant possédant l'activité phytase issu de l'étape (d)
(f) purification de la phytase à partir du surnageant récupéré à l'étape (e)
(g) incorporation de la phytase purifiée à l'étape (f) dans ladite composition alimentaire.

43. Procédé de production d'une composition alimentaire selon la revendication 33, **caractérisé en ce qu'**il comprend les étapes de :
(a) mise en culture d'un organisme hôte transformé selon l'une des revendications 12 à 18
(b) récupération du milieu de culture par élimination dudit organisme hôte transformé
(c) purification de la phytase à partir du milieu de culture récupéré à l'étape (b)
(d) incorporation de la phytase purifiée à l'étape (c) dans ladite composition alimentaire.

44. Procédé pour augmenter l'assimilation du phosphate inorganique contenu dans l'acide phytique des aliments à base de végétaux par les animaux monogastriques, **caractérisé en ce que** l'on incorpore une phytase selon l'une des revendications 5 à 7 ou une composition enzymatique selon l'une des revendications 27 et 28 dans l'alimentation desdits animaux.

45. Procédé selon la revendication 44, **caractérisé en ce que** l'on alimente lesdits animaux monogastriques avec une composition alimentaire selon l'une des revendications 29 à 33.

46. Procédé pour diminuer l'adjonction de phosphore dans l'alimentation des animaux monogastriques, **caractérisé en ce que** l'on alimente lesdits animaux avec une composition alimentaire selon l'une des revendications 29 à 33.

47. Procédé pour diminuer les rejets de phosphore issus de l'alimentation des animaux monogastriques, **caractérisé en ce que** l'on alimente lesdits animaux avec une composition alimentaire selon l'une des revendications 29 à 33.

## Claims

1. Isolated polynucleotide encoding a phytase, **characterized in that** it is selected from the group consisting of:
(a) an isolated polynucleotide encoding the phytase described by the sequence identifier SEQ ID NO: 2
(b) an isolated polynucleotide which hybridizes with the polynucleotide according to (a) under stringent conditions
(c) an isolated polynucleotide which is at least 70% identical to the polynucleotide according to (a)
said phytase having an optimum pH for activity of less than or equal to 4

2. Polynucleotide according to Claim 1, **characterized in that** it is represented by the sequence identifier SEQ ID NO: 1.

3. Polynucleotide according to either of Claims 1 and 2, **characterized in that** it originates from a bacterium of the *Acidocella* genus.

4. Isolated polynucleotide comprising a polynucleotide according to one of Claims 1 to 3.

5. Phytase, **characterized in that** it is encoded by a polynucleotide according to one of claims 1 to 4, said phytase having an optimum pH for activity of less than or equal to 4.

6. Phytase according to Claim 5, **characterized in that** it is selected from the group consisting of:
(a) the phytase represented by the sequence identifier SEQ ID NO: 2
(b) a phytase which is at least 70% identical to the phytase according to (a)
(c) a biologically active fragment of a phytase according to (a) or (b).

7. Phytase according to either of Claims 5 or 6, **characterized in that** it originates from a bacterium of the *Acidocella* genus.

8. Chimeric gene comprising at least, functionally linked to one another:
(a) a promoter which is functional in a host organism
(b) a polynucleotide according to one of Claims 1 to 4
(c) a terminator element which is functional in host organism.

9. Chimeric gene according to Claim 8, **characterized in that** it also comprises a signal peptide or a transit peptide which is functional in said host organism.

10. Expression or transformation vector containing a chimeric gene according to either of Claims 8 and 9.

11. Vector according to Claim 10, **characterized in that** it is a plasmid, a phage or a virus.

12. Transformed host organism containing a vector according to Claim 10.

13. Host organism according to Claim 12, **characterized in that** it is a microorganism.

14. Host organism according to Claim 13, **characterized in that** the microorganism is selected from bacteria, fungi, yeast and viruses.

15. Host organism according to Claim 14, **characterized in that** the microorganism is a bacterium selected from the *Corynebacterium, Streptomyces* and *Escherichia* genera, in particular *E. coli.*

16. Host organism according to Claim 14, **characterized in that** the microorganism is a fungus selected from the *Penicillium, Aspergillus, Chrysosporium* and *Trichoderma* genera.

17. Host organism according to Claim 14, **characterized in that** the microorganism is a yeast selected from the *Saccharomyces, Kluyveromyces* and *Pichia* genera.

18. Host organism according to Claim 12, **characterized in that** it is a plant cell, a plant or a part of a plant.

19. Bacterial extract comprising at least one phytase activity, **characterized in that** it originates from a bacterium of the *Acidocella* genus and of which the optimum pH for activity of said phytase activity is less than or equal to 4.

20. Bacterial extract according to Claim 19, **characterized in that** the phytase activity has the following properties:
(a) optimum temperature = 70°C
(b) optimum pH = 4.

21. Bacterial extract according to Claim 19, **characterized in that** the phytase activity has the following properties:
(a) optimum temperature = 60°C
(b) optimum pH = 3.5.

22. Method for preparing a bacterial extract according to one of Claims 19 to 21, **characterized in that** it comprises the steps of:
(a) culturing a bacterial strain of the *Acidocella* genus
(b) concentrating the bacteria cultured in step (a)
(c) grinding the bacteria isolated in step (b)
(d) centrifuging the ground material obtained in step (c)
(e) recovering the supernatant having the phytase activity derived from step (d).

23. Method for producing a phytase according to one of Claims 5 to 7, **characterized in that** it comprises the steps of:
(a) culturing a bacterial strain of the *Acidocella* genus
(b) concentrating the bacteria cultured in step (a)
(c) grinding the bacteria isolated in step (b)
(d) centrifuging the ground material obtained in step (c)
(e) recovering the supernatant having the phytase activity derived from step (d)
(f) purifying the phytase from the supernatant recovered in step (e).

24. Method for producing a phytase according to one of Claims 5 to 7, **characterized in that** it comprises the steps of:
(a) culturing a bacterial strain of the *Acidocella* genus
(b) recovering the culture medium by removing the bacteria
(c) purifying the phytase from the culture medium recovered in step (b).

25. Method for producing a phytase according to one of Claims 5 to 7, **characterized in that** it comprises the steps of:
(a) culturing a transformed host organism according to one of Claims 12 to 18
(b) concentrating the transformed host organism cultured in step (a)
(c) grinding the transformed host organism isolated in step (b)
(d) centrifuging the ground material obtained in step (c)
(e) recovering the supernatant having the phytase activity derived from step (d)
(f) purifying the phytase from the supernatant recovered in step (e).

26. Method for producing a phytase according to one of Claims 5 to 7, **characterized in that** it comprises the steps of:
(a) culturing a transformed host organism according to one of claims 12 to 18
(b) recovering the culture medium by removing said transformed host organism
(c) purifying the phytase from the culture medium recovered in step (b).

27. Enzymatic composition, **characterized in that** it comprises at least one bacterial extract according to one of Claims 19 to 21.

28. Enzymatic composition, **characterized in that** it comprises at least one phytase according to one of Claims 5 to 7.

29. Feed composition, **characterized in that** it comprises at least one transformed host organism according to one of Claims 12 to 18.

30. Feed composition, **characterized in that** it comprises at least one bacterium of the *Acidocella* genus expressing a phytase according to one of Claims 5 to 7.

31. Feed composition according to Claim 30, **characterized in that** the bacterium is selected from the species *Acidocella aminolytica* and *Acidocella facilis*.

32. Feed composition, **characterized in that** it comprises at least one bacterial extract according to one of Claims 19 to 21.

33. Feed composition, **characterized in that** it comprises at least one phytase according to one of claims 5 to 7.

34. Use of a feed composition according to one of Claims 29 to 33, for monogastric animal nutrition.

35. Use of a feed composition according to Claim 34, **characterized in that** it is intended for pig nutrition.

36. Use of a feed composition according to Claim 34, **characterized in that** it is intended for poultry nutrition.

37. Method for producing a feed composition according to Claim 33, **characterized in that** it comprises the steps of:
(a) culturing a host organism according to one of claims 12 to 18
(b) concentrating the host organism cultured in step (a)
(c) incorporating the host organism isolated in step (b) into said feed composition.

38. Method for producing a feed composition according to either of Claims 30 and 31, **characterized in that** it comprises the steps of:
(a) culturing a bacterial strain of the *Acidocella* genus
(b) concentrating the bacteria cultured in step (a)
(c) incorporating the bacteria isolated in step (b) into said feed composition.

39. Method for producing a feed composition according to Claim 32, **characterized in that** it comprises the steps of:
(a) culturing a bacterial strain of the *Acidocella* genus
(b) concentrating the bacteria cultured in step (a)
(c) grinding the bacteria isolated in step (b)
(d) centrifuging the ground material obtained in step (c)
(e) recovering the supernatant having the phytase activity derived from step (d).
(f) incorporating the supernatant recovered in step (e) into said feed composition.

40. Method for producing a feed composition according to Claim 33, **characterized in that** it comprises the steps of:
(a) culturing a bacterial strain of the *Acidocella* genus
(b) concentrating the bacteria cultured in step (a)
(c) grinding the bacteria isolated in step (b)
(d) centrifuging the ground material obtained in step (c)
(e) recovering the supernatant having the phytase activity derived from step (d)
(f) purifying the phytase from the supernatant recovered in step (e)
(g) incorporating the phytase purified in step (f) into said feed composition.

41. Method for producing a feed composition according to Claim 33, **characterized in that** it comprises the steps of:
(a) culturing a bacterial strain of the *Acidocella* genus
(b) recovering the culture medium by removing the bacteria
(c) purifying the phytase from the culture medium recovered in step (b)
(d) incorporating the phytase purified in step (c) into said feed composition.

42. Method for producing a feed composition according to Claim 33, **characterized in that** it comprises the steps of:
(a) culturing a transformed host organism according to one of Claims 12 to 18
(b) concentrating the transformed host organism cultured in step (a)
(c) grinding the transformed host organism isolated in step (b)
(d) centrifuging the ground material obtained in step (c)
(e) recovering the supernatant having the phytase activity derived from step (d)
(f) purifying the phytase from the supernatant recovered in step (e)
(g) incorporating the phytase purified in step (f) into said feed composition.

43. Method for producing a feed composition according to Claim 33, **characterized in that** it comprises the steps of:
(a) culturing a transformed host organism according to one of Claims 12 to 18
(b) recovering the culture medium by removing said transformed host organism
(c) purifying the phytase from the culture medium recovered in step (b)
(d) incorporating the phytase purified in step (c) into said feed composition.

44. Method for increasing the assimilation of the inorganic phosphate contained in the phytic acid of plant-based feedstuffs by monogastric animals, **characterized in that** a phytase according to one of Claims 5 to 7 or an enzymatic composition according to either of Claims 27 and 28 is incorporated into the nutrition of said animals.

45. Method according to Claim 44, **characterized in that** said monogastric animals are fed with a feed composition according to one of Claims 29 to 33.

46. Method for decreasing the addition of phosphorus to monogastric animal nutrition, **characterized in that** said animals are fed with a feed composition according to one of Claims 29 to 33.

47. A method for decreasing the phosphorus waste derived from monogastric animal nutrition, **characterized in that** said animals are fed with a feed composition according to one of Claims 29 to 33.

## Patentansprüche

1. Isoliertes Polynukleotid, das für eine Phytase codiert, **dadurch gekennzeichnet, dass** es aus der folgenden Gruppe ausgewählt ist:
(a) isoliertes Polynukleotid, das für die Phytase codiert, die von der Sequenz mit der Beschreibung SEQ ID Nr.: 2 beschrieben wird,
(b) isoliertes Polynukleotid, das unter stringenten Bedingungen mit dem Polynukleotid gemäß (a) hybridisiert,
(c) isoliertes Polynukleotid mit mindestens 70% Identität zu dem Polynukleotid gemäß (a),
wobei die Phytase einen optimalen Aktivitäts-pH von 4 oder darunter aufweist.

2. Polynukleotid nach Anspruch 1, **dadurch gekennzeichnet, dass** es von der Sequenz mit der Beschreibung SEQ ID Nr.: 1 angegeben wird.

3. Polynukleotid nach Anspruch 1 oder 2, **dadurch gekennzeichnet, dass** es von einem Bakterium der Gattung *Acidocella* stammt.

4. Isoliertes Polynukleotid, das ein Polynukleotid nach einem der Ansprüche 1 bis 3 umfasst.

5. Phytase, **dadurch gekennzeichnet, dass** sie von einem Polynukleotid nach einem der Ansprüche 1 bis 4 codiert wird, wobei die Phytase einen optimalen Aktivitäts-pH von 4 oder darunter aufweist.

6. Phytase nach Anspruch 5, **dadurch gekennzeichnet, dass** sie aus der folgenden Gruppe ausgewählt ist:
(a) Phytase, die von der Sequenz mit der Beschreibung SEQ ID Nr.: 2 angegeben wird
(b) Phytase mit mindestens 70% Identität zu der Phytase gemäß (a)
(c) biologisch aktives Fragment einer Phytase gemäß (a) oder (b).

7. Phytase nach Anspruch 5 oder 6, **dadurch gekennzeichnet, dass** sie von einem Bakterium der Gattung *Acidocella* stammt.

8. Chimäres Gen, das operativ verknüpft mindestens Folgendes umfasst:
(a) einen in einem Wirtsorganismus funktionsfähigen Promoter
(b) ein Polynukleotid nach einem der Ansprüche 1 bis 4
(c) ein in einem Wirtsorganismus funktionsfähiges Terminatorelement.

9. Chimäres Gen nach Anspruch 8, **dadurch gekennzeichnet, dass** es weiterhin ein in dem Wirtsorganismus funktionsfähiges Signalpeptid oder Transitpeptid umfasst.

10. Expressions- oder Transformationsvektor, der ein chimäres Gen nach Anspruch 8 oder 9 enthält.

11. Vektor nach Anspruch 10, **dadurch gekennzeichnet, dass** es sich um ein Plasmid, einen Phagen oder ein Virus handelt.

12. Transformierter Wirtsorganismus, der einen Vektor nach Anspruch 10 enthält.

13. Wirtsorganismus nach Anspruch 12, **dadurch gekennzeichnet, dass** es sich um einen Mikroorganismus handelt.

14. Wirtsorganismus nach Anspruch 13, **dadurch gekennzeichnet, dass** der Mikroorganismus aus der Reihe Bakterien, Pilze, Hefen oder Viren ausgewählt ist.

15. Wirtsorganismus nach Anspruch 14, **dadurch gekennzeichnet, dass** es sich bei dem Mikroorganismus um ein Bakterium ausgewählt aus den Gattungen *Corynebacterium, Streptomyces* und *Escherichia,* insbesondere *E. coli,* handelt.

16. Wirtsorganismus nach Anspruch 14, **dadurch gekennzeichnet, dass** es sich bei dem Mikroorganismus um einen Pilz ausgewählt aus den Gattungen *Penicillium, Aspergillus, Chrysosporium* und *Trichoderma* handelt.

17. Wirtsorganismus nach Anspruch 14, **dadurch gekennzeichnet, dass** es sich bei dem Mikroorganismus um eine Hefe ausgewählt aus den Gattungen *Saccharomyces, Kluyveromyces* und *Pichia* handelt.

18. Wirtsorganismus nach Anspruch 12, **dadurch gekennzeichnet, dass** es sich um eine Pflanzenzelle, eine Pflanze oder einen Pflanzenteil handelt.

19. Bakterienextrakt, der mindestens eine Phytaseaktivität enthält, **dadurch gekennzeichnet, dass** er von einem Bakterium der Gattung *Acidocella* stammt und dass sein optimaler Aktivitäts-pH der Phytaseaktivität gleich 4 oder darunter beträgt.

20. Bakterienextrakt nach Anspruch 19, **dadurch gekennzeichnet, dass** die Phytaseaktivität die folgenden Eigenschaften aufweist:
(a) Temperaturoptimum = 70°C
(b) pH-Optimum = 4.

21. Bakterienextrakt nach Anspruch 19, **dadurch gekennzeichnet, dass** die Phytaseaktivität die folgenden Eigenschaften aufweist:
(a) Temperaturoptimum = 60°C
(b) pH-Optimum = 3,5.

22. Verfahren zur Herstellung eines Bakterienextrakts nach einem der Ansprüche 19 bis 21, **dadurch gekennzeichnet, dass** es die folgenden Schritte umfasst:
(a) Kultivieren eines Bakterienstamms der Gattung *Acidocella*
(b) Aufkonzentrieren der in Schritt (a) gezüchteten Bakterien
(c) Aufbrechen der in Schritt (b) isolierten Bakterien
(d) Zentrifugieren des in Schritt (c) erhaltenen Aufbruchprodukts
(e) Gewinnen des in Schritt (d) erhaltenen Überstands mit der Phytaseaktivität.

23. Verfahren zur Herstellung einer Phytase nach einem der Ansprüche 5 bis 7, **dadurch gekennzeichnet, dass** es die folgenden Schritte umfasst:
(a) Kultivieren eines Bakterienstamms der Gattung *Acidocella*
(b) Aufkonzentrieren der in Schritt (a) gezüchteten Bakterien
(c) Aufbrechen der in Schritt (b) isolierten Bakterien
(d) Zentrifugieren des in Schritt (c) erhaltenen Aufbruchprodukts
(e) Gewinnen des in Schritt (d) erhaltenen Überstands mit der Phytaseaktivität
(f) Aufreinigen der Phytase aus dem in Schritt (e) gewonnenen Überstand.

24. Verfahren zur Herstellung einer Phytase nach einem der Ansprüche 5 bis 7, **dadurch gekennzeichnet, dass** es die folgenden Schritte umfasst:
(a) Kultivieren eines Bakterienstamms der Gattung *Acidocella*
(b) Gewinnen des Kulturmediums durch Entfernen der Bakterien
(c) Aufreinigen der Phytase aus dem in Schritt (b) gewonnenen Kulturmedium.

25. Verfahren zur Herstellung einer Phytase nach einem der Ansprüche 5 bis 7, **dadurch gekennzeichnet, dass** es die folgenden Schritte umfasst:
(a) Kultivieren eines transformierten Wirtsorganismus nach einem der Ansprüche 12 bis 18
(b) Aufkonzentrieren des in Schritt (a) gezüchteten transformierten Wirtsorganismus
(c) Aufbrechen des in Schritt (b) isolierten transformierten Wirtsorganismus
(d) Zentrifugieren des in Schritt (c) erhaltenen Aufbruchprodukts
(e) Gewinnen des in Schritt (d) gewonnenen Überstands mit der Phytaseaktivität
(f) Aufreinigen der Phytase aus dem in Schritt (e) gewonnenen Überstand.

26. Verfahren zur Herstellung einer Phytase nach einem der Ansprüche 5 bis 7, **dadurch gekennzeichnet, dass** es die folgenden Schritte umfasst:
(a) Kultivieren eines transformierten Wirtsorganismus nach einem der Ansprüche 12 bis 18
(b) Gewinnen des Kulturmediums durch Entfernen des transformierten Wirtsorganismus
(c) Aufreinigen der Phytase aus dem in Schritt (b) gewonnenen Kulturmedium.

27. Enzymzusammensetzung, **dadurch gekennzeichnet, dass** sie mindestens einen Bakterienextrakt nach einem der Ansprüche 19 bis 21 umfasst.

28. Enzymzusammensetzung, **dadurch gekennzeichnet, dass** sie mindestens eine Phytase nach einem der Ansprüche 5 bis 7 umfasst.

29. Enzymzusammensetzung, **dadurch gekennzeichnet, dass** sie mindestens einen transformierten Wirtsorganismus nach einem der Ansprüche 12 bis 18 umfasst.

30. Futtermittelzusammensetzung, **dadurch gekennzeichnet, dass** sie mindestens ein Bakterium der Gattung *Acidocella,* das eine Phytase nach einem der Ansprüche 5 bis 7 exprimiert, umfasst.

31. Futtermittelzusammensetzung nach Anspruch 30, **dadurch gekennzeichnet, dass** das Bakterium aus den Arten *Acidocella aminolytica* und *Acidocella facilis* ausgewählt ist.

32. Futtermittelzusammensetzung, **dadurch gekennzeichnet, dass** sie mindestens einen Bakterienextrakt nach einem der Ansprüche 19 bis 21 umfasst.

33. Futtermittelzusammensetzung, **dadurch gekennzeichnet, dass** sie mindestens eine Phytase nach einem der Ansprüche 5 bis 7 umfasst.

34. Verwendung einer Futtermittelzusammensetzung nach einem der Ansprüche 29 bis 33 für die Fütterung von Monogastriern.

35. Verwendung einer Futtermittelzusammensetzung nach Ansprüche 34, **dadurch gekennzeichnet, dass** sie für die Fütterung von Schweinen bestimmt ist.

36. Verwendung einer Futtermittelzusammensetzung nach Anspruch 34, **dadurch gekennzeichnet, dass** sie für die Fütterung von Geflügel bestimmt ist.

37. Verfahren zur Herstellung einer Futtermittelzusammensetzung nach Anspruch 33, **dadurch gekennzeichnet, dass** es die folgenden Schritte umfasst:
(a) Kultivieren eines Wirtsorganismus nach einem der Ansprüche 12 bis 18
(b) Aufkonzentrieren des in Schritt (a) gezüchteten Wirtsorganismus
(c) Einarbeiten des in Schritt (b) isolierten Wirtsorganismus in die Futtermittelzusammensetzung.

38. Verfahren zur Herstellung einer Futtermittelzusammensetzung nach Anspruch 30 oder 31, **dadurch gekennzeichnet, dass** es die folgenden Schritte umfasst:
(a) Kultivieren eines Bakterienstamms der Gattung *Acidocella*
(b) Aufkonzentrieren der in Schritt (a) gezüchteten Bakterien
(c) Einarbeiten der in Schritt (b) isolierten Bakterien in die Futtermittelzusammensetzung.

39. Verfahren zur Herstellung einer Futtermittelzusammensetzung nach Anspruch 32, **dadurch gekennzeichnet, dass** es die folgenden Schritte umfasst:
(a) Kultivieren eines Bakterienstamms der Gattung *Acidocella*
(b) Aufkonzentrieren der in Schritt (a) gezüchteten Bakterien
(c) Aufbrechen der in Schritt (b) isolierten Bakterien
(d) Zentrifugieren des in Schritt (c) erhaltenen Aufbruchprodukts
(e) Gewinnen des in Schritt (d) erhaltenen Überstands mit der Phytaseaktivität
(f) Einarbeiten des in Schritt (e) gewonnenen Überstands in die Futtermittelzusammensetzung.

40. Verfahren zur Herstellung einer Futtermittelzusammensetzung nach Anspruch 33, **dadurch gekennzeichnet, dass** es die folgenden Schritte umfasst:
(a) Kultivieren eines Bakterienstamms der Gattung *Acidocella*
(b) Aufkonzentrieren der in Schritt (a) gezüchteten Bakterien
(c) Aufbrechen der in Schritt (b) isolierten Bakterien
(d) Zentrifugieren des in Schritt (c) erhaltenen Aufbruchprodukts
(e) Gewinnen des in Schritt (d) erhaltenen Überstands mit der Phytaseaktivität
(f) Aufreinigen der Phytase aus dem in Schritt (e) gewonnenen Überstand
(g) Einarbeiten der in Schritt (f) gereinigten Phytase in die Futtermittelzusammensetzung.

41. Verfahren zur Herstellung einer Futtermittelzusammensetzung nach Anspruch 33, **dadurch gekennzeichnet, dass** es die folgenden Schritte umfasst:
(a) Kultivieren eines Bakterienstamms der Gattung *Acidocella*
(b) Gewinnen des Kulturmediums durch Entfernen der Bakterien
(c) Aufreinigen der Phytase aus dem in Schritt (b) gewonnenen Kulturmedium
(d) Einarbeiten der in Schritt (c) gereinigten Phytase in die Futtermittelzusammensetzung.

42. Verfahren zur Herstellung einer Futtermittelzusammensetzung nach Anspruch 33, **dadurch gekennzeichnet, dass** es die folgenden Schritte umfasst:
(a) Kultivieren eines transformierten Wirtsorganismus nach einem der Ansprüche 12 bis 18
(b) Aufkonzentrieren des in Schritt (a) gezüchteten transformierten Wirtsorganismus
(c) Aufbrechen des in Schritt (b) isolierten transformierten Wirtsorganismus
(d) Zentrifugieren des in Schritt (c) erhaltenen Aufbruchprodukts
(e) Gewinnen des in Schritt (d) gewonnenen Überstands mit der Phytaseaktivität
(f) Aufreinigen der Phytase aus dem in Schritt (e) gewonnenen Überstand
(g) Einarbeiten der in Schritt (f) gereinigten Phytase in die Futtermittelzusammensetzung.

43. Verfahren zur Herstellung einer Futtermittelzusammensetzung nach Anspruch 33, **dadurch gekennzeichnet, dass** es die folgenden Schritte umfasst:
(a) Kultivieren eines transformierten Wirtsorganismus nach einem der Ansprüche 12 bis 18
(b) Gewinnen des Kulturmediums durch Entfernen des transformierten Wirtsorganismus
(c) Aufreinigen der Phytase aus dem in Schritt (b) gewonnenen Kulturmedium
(d) Einarbeiten der in Schritt (c) gereinigten Phytase in die Futtermittelzusammensetzung.

44. Verfahren zum Erhöhen der Aufnahme des in der Phytinsäure von pflanzlichen Futtermitteln enthaltenen anorganischen Phosphats durch Monogastrier, **dadurch gekennzeichnet, dass** man eine Phytase nach einem der Ansprüche 5 bis 7 oder eine Enzymzusammensetzung nach Anspruch 27 oder 28 in das Futter dieser Tiere einarbeitet.

45. Verfahren nach Anspruch 44, **dadurch gekennzeichnet, dass** man die Monogastrier mit einer Futtermittelzusammensetzung nach einem der Ansprüche 29 bis 33 füttert.

46. Verfahren zur Verringerung der Zugabe von Phosphor in das Futter von Monogastriern, **dadurch gekennzeichnet, dass** man die Tiere mit einer Futtermittelzusammensetzung nach einem der Ansprüche 29 bis 33 füttert.

47. Verfahren zur Verringerung der Phosphorausscheidungen aus der Fütterung von Monogastriern, **dadurch gekennzeichnet, dass** man die Tiere mit einer Futtermittelzusammensetzung nach einem der Ansprüche 29 bis 33 füttert.
